# EUROPEAN PATENT APPLICATION

(11) **EP 3 388 439 A1**
(43) Date of publication of application: **17.10.2018**
(21) Application number: 17165939.4
(22) Date of filing: 11.04.2017
(51) Int. Cl.: C07D 495/04, C08B 37/10, C08L 5/10, A61K 31/727, A61P 35/00

(54) **BIOTIN-CONJUGATED N-ACETYL GLYCOL SPLIT HEPARIN**

(71) Applicant: Leadiant Biosciences SA, 6850 Mendrisio (CH)
(72) Inventor: GIANNINI, Giuseppe, 00071 Pomezia (ROMA) (IT); NAGGI, Annamaria, 20025 Legnano (MI) (IT); ESPOSITO, Emiliano, 24047 Treviglio (BG) (IT)
(74) Representative: Ghirardi, Valeria

(57) **Abstract**

The present invention relates to N-acetyl glycol split heparin molecules conjugated with biotin having the following formula (I) 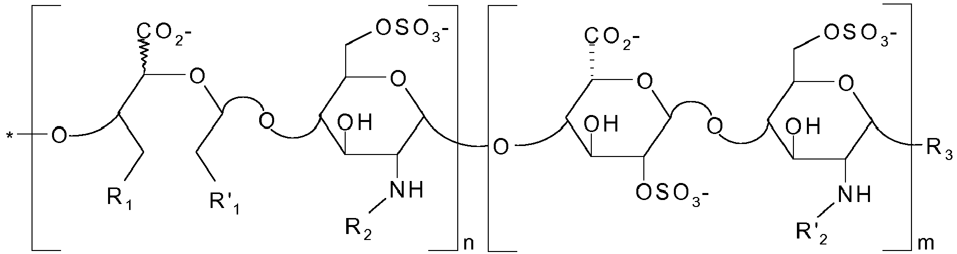 wherein the meanings of the substituents are disclosed in the description.

The present invention also relates to such compounds for use as medicaments, in particular for the treatment of diseases and disorders associated with heparanase activity, and to pharmaceutical compositions comprising the same.

## Description

### FIELD OF THE INVENTION

The present invention relates to N-acetyl glycol split heparin molecules conjugated with biotin.

The present invention also relates to such compounds for use as medicaments, in particular for the treatment of diseases and disorders associated with heparanase activity, and to pharmaceutical compositions comprising the same.

### BACKGROUND OF THE INVENTION

Heparan sulfate proteoglycans (HSPGs) are major components of the basement membrane and extracellular matrix. They are ubiquitous macromolecules associated with the cell surface and extracellular matrix (ECM) of a wide range of cells of vertebrate and invertebrate tissues (Bernfield et al. Annu. Rev. Biochem. 1999, 68, 729; lozzo R. V., San Antonio J. D. J. Clin. Invest. 2001, 108, 349; Kjellen L., Lindahl U. Annu. Rev. Biochem. 1991, 60, 443). HSPGs are a class of carbohydrate-modified proteins involved in key biological processes, including a role as co-receptors for a number of ligand molecules to regulate their signaling and distribution (Nakato H., Li J.P.. Int. Rev. Cell. Mol. Biol. 2016, 325, 275).

Heparan sulfate (HS) is a component of cell surface and matrix-associated proteoglycans (HSPGs). A key function of HS is to bind and interact with signaling proteins, growth factors, plasma proteins, immune-modulators and other factors. In doing so, the HS chains and HSPGs are able to regulate protein distribution, bio-availability and action on target cells. Moreover, the HS chains ensure that a wide variety of bioactive molecules bind to the cell surface and ECM and thereby function in the control of normal and pathological processes, among which are morphogenesis, tissue repair, inflammation, vascularization and cancer metastasis (Billings P.C., Pacifici M. Connect Tissue Res. 2015, 56(4), 272). HS glycosaminoglycans bind to and assemble a multitude of ECM proteins (i.e., laminin, fibronectin, collagen type IV) and thereby significantly contribute to the ECM self-assembly and integrity.

Heparanase is an endoglycosidase which cleaves heparan sulfate (HS) and hence participates in degradation and remodeling of the extracellular matrix (ECM), with a release of bioactive saccharide fragments and HS-bound growth factors and cytokines. Heparanase is preferentially expressed in human tumors and its over-expression in tumor cells confers an invasive phenotype in experimental animals. Heparanase upregulation correlates with increased tumor vascularity and poor postoperative survival of cancer patients. Heparanase is synthesized as a 65 kDa inactive precursor that undergoes proteolytic cleavage, yielding 8 kDa and 50 kDa protein subunits that heterodimerize to form an active enzyme.

Heparanase exhibits also non-enzymatic activities, independent of its involvement in ECM degradation.

There is growing evidence that heparanase upregulates expression of genes that participate in cancer metastasis and angiogenesis, glucose metabolism, immune response, inflammation and atherosclerosis, suggesting that heparanase belongs to an emerging class of proteins that play a significant role in regulating transcription in addition to their well-recognized extra-nuclear functions (Pisano C. et al. Biochem. Pharmacol. 2014, 89, 12; Ilan N. et al. Int. J. Biochem. Cell Biol. 2006, 38, 2018; Fux L. et al. Trends Biochem. Sci. 2009, 34, 511; Parish C.R.et al. Matrix Biol. 2013, 32, 228; Ramani V.C. et al. FEBS J. 2013, 280, 2294; Vlodavsky I. et al. Matrix Biol. 2013. 32, 241; Yang Y. et al. Cancer Res. 2010, 70, 8329).

In recent years, heparanase has attracted considerable attention as a promising target for innovative pharmacological applications. Indeed, heparanase appears to be involved in major human diseases, from tumors to chronic inflammation, diabetic nephropathy, bone osteolysis, thrombosis and atherosclerosis, in addition to more recent investigation in various rare diseases (Rivara S., Milazzo F.M., Giannini G.. Future Med Chem. 2016; 8(6), 647-80. Heparanase: a rainbow pharmacological target associated to multiple pathologies including rare diseases).

In view of the above, compounds able to specifically modulate the activity of this enzyme are highly desired as a useful pharmacological option for many therapeutic indications. Since heparanase is involved in a very wide range of molecular pathways, modulation of the expression or activity of this enzyme is a highly viable therapeutic option.

Some compounds are known in the art as heparanase inhibitors. In particular, three classes of heparanase inhibitors are known: active analogous of endogenous substance, synthetic small molecule compounds and natural products. In the literature, monoclonal antibodies and nucleic acid-based inhibitors of heparanase are also mentioned.

Among the most promising heparanase-inhibiting drug is N-desulfated, N-acetylated glycol split (gs) heparin (SST0001, also named as Roneparstat; Leadiant Bioscience S.A.; former Sigma-Tau Research, Switzerland), possessing anti-tumor activity arising from its high heparanase-inhibiting activity (Barbieri, P., Paoletti, D., Giannini, G., Sanderson, R.D., Noseda, A. J. Pharmacol. Clin. Toxicol. 2017, 5(2), 1071. Roneparstat and Heparanase Inhibition: A New Tool for Cancer Treatment. Ritchie, J. P., Ramani, V. C., Ren, Y., Naggi, A., Torri, G., Casu, B., et al. Clinical Cancer Research, 2011, 17, 1382-1393. SST0001, a chemically modified heparin: Inhibits myeloma growth and angiogenesis via disruption of the heparanase/syndecan-1 axis.). Roneparstat is a heparin derivative obtained by periodate oxidation/borohydride reduction of totally N-desulfated and N-acetylated heparin. This compound is disclosed in EP2343077, US7781416 and US8067555.

Besides its useful activities the use of Roneparstat has some disadvantages.

The main disadvantage of Roneparstat is the difficulty of neutralize its activity in case of over dosages or intolerance.

In particular, it would be desirable to have a compound for which antidotes able to quickly neutralize its activity are available.

Also, Roneparstat and similar heparin derivatives are eliminated from the plasma in a relatively rapid manner; therefore administration by infusion would be preferred with respect to repeated subcutaneous administrations (Yang, Y., MacLeod, V., Dai, Y., Khotskaya-Sample, Y., Shriver, Z., Venkataraman, G., Sasisekharan, R., Naggi, A., Torri, G.,,Casu, B., Vlodavsky, I., Suva, L.J., Epstein, J., Yaccoby, S., Shaughnessy, J.D., Barlogie, B., Sanderson, R.D.. Blood 2007, 110(6), 2041-8. The syndecan-1 heparan sulfate proteoglycan is a viable target for myeloma therapy). However, this kind of administration can be uncomfortable for the patient especially for routine treatments. Therefore, Roneparstat and similar derivatives are usually administered subcutaneously and this limits their bioavailability.

Therefore, it is desirable to modify the structure of Roneparstat in order to increase its bioavailability. However, modifications to its structure can easily alter its heparanase inhibitory activity.

Therefore, there is the need of a modified version of Roneparstat able to overcome the above mentioned disadvantages while at the same time maintaining and not impairing its activity of inhibition of heparanase.

### SUMMARY OF THE INVENTION

It has now been found that a compound derived from N-desulfated, N-acetylated glycol split heparin characterized by the presence of a biotin moiety is able to overcome the above mentioned disadvantages at the same time maintaining its efficacy as heparanase inhibitor.

In particular, the biotin moiety can be linked to the heparin structure through a suitable linker comprising functional groups, which modify some properties of the molecule, such as hydrophilicity, thus improving its bioavailability.

More importantly, the biotin moieties allow an easier drug activity neutralization by administration of avidine, as the affinity between avidine and biotin is one of the strongest known in nature.

Therefore, the presence of biotin is particularly useful if a rapid neutralization of the drug in the blood is desired, for example in cases of over dosage or intolerance, thanks to the presence of a specific and immediately active reversal agent (avidine), which can therefore be used as antidote. At this end, it is also noteworthy that the biotin moieties in the compounds of the invention can still be recognized by avidine.

The biotin moieties, also, provide functional groups easily detectable by, for example, interaction tests with avidine, thus allowing an easier monitoring of plasmatic concentrations of the molecule.

Importantly, the compounds of the invention, notwithstanding such modifications, maintain the inhibitory activity of heparanase, which is required for their therapeutic applications.

It is an object of the present invention a compound having the following formula (I) 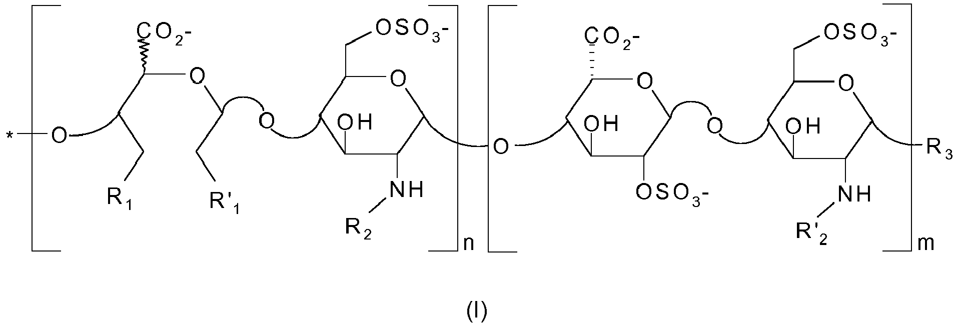 wherein

in each n unit R₁ and R₁' are independently selected from OH and R₄-biotin, wherein R₄ is NH or NH-NH or HN-C₁C₆₀-alkylene-NH,
wherein
in said alkylene chain one or more CH₂ group is optionally substituted by one or more of the following groups: O, NH and CO and
in said alkylene chain a hydrogen of one or more CH₂ group is optionally substituted by the side chain of a D or L amino acid;

in each n and m unit R₂ and R₂' are independently selected from an acetyl group, biotin and R₅-biotin, wherein R₅ is CO-C₁C₆₀-alkylene-NH,
wherein in said alkylene chain one or more CH₂ group is optionally substituted by one or more of the following groups: O, NH and CO and
in said alkylene chain a hydrogen of one or more CH₂ group is optionally substituted by the side chain of a D or L amino acid;

R₃ is selected from the group consisting of OH and a disaccharidic moiety of the following formula (II) 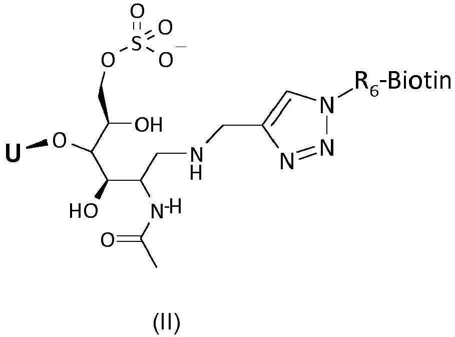
wherein U is selected from 20-sulfated uronic acid and glycol-split uronic acid,
and R₆ is NH or NH-NH or C₁C₆₀-alkylene-NH,
   wherein in said alkylene chain one or more CH₂ group is optionally substituted by one or more of the following groups: O, NH and CO and
   in said alkylene chain a hydrogen of one or more CH₂ group is optionally substituted by the side chain of a D or L amino acid;
n and m, which can be the same or different, may vary from 1 to 40; the sum of n+m ranges from 6 to 40; the m:n ratio ranges from 10:2 to 1:1, and the units marked with m and n are statistically distributed along the polysaccharide chain and are not necessarily in sequence,
wherein at least one of said groups biotin, R₄-biotin, R₅-biotin and R₆-biotin is present with a substitution degree, expressed in percentage of substituent groups with respect to the total of disaccharide units, comprised between 1 and 50%,
the enantiomers, diastereoisomers, and mixtures thereof,
or a pharmaceutically acceptable salt, hydrate or solvate thereof.

In a preferred embodiment, said substitution degree is comprised between 3 and 10%.

In a preferred embodiment, said compound of formula (I) comprises at least one R₅-biotin group in position R₂ and/or R₂' wherein said R₅ comprises a polyethylene glycol (PEG).

In a preferred embodiment, said compound of formula (I) comprises at least one R₄-biotin group in position R₁ and/or R₁' wherein said R₄ is or comprises an amino acid chain, preferably it comprises an amino acid chain and a cadaverine residue.

In a preferred embodiment, said compound of formula (I) comprises at least one R₆-biotin group in position R₃ wherein said R₆ is or comprises a glycine residue and a pentylamine residue in any order.

Processes for the preparation of the compound of formula (I), as will be better defined below, are also objects of the present invention.

It is also an object of the invention the compound of formula (I) for use as a medicament.

A further object of the present invention is the compound of formula (I) for use in the treatment of a disease which can be improved or prevented by an anti-heparanase activity.

In a preferred embodiment, said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging.

A further object of the invention is a pharmaceutical composition containing as active ingredient a compound of formula (I) and at least one pharmaceutically acceptable vehicle and/or excipient.

### Detailed description of the invention

### Figures

Figure 1. Antimetastatic activity of roneparstat biotinylated derivatives in the lungs of mice, in a B16BL6 experimental metastatic tumor model.
Figure 2. Inhibition of CAG myeloma cell growth of biotinylated glycol-split heparins versus SST0001 (Roneparstat).

### Definitions

Within the meaning of the present invention, the term "anti-heparanase activity" refers to an activity sufficient to inhibit partially or totally the enzyme heparanase.

Within the meaning of the present invention, biotin is a water-soluble B-vitamin, also called vitamin B7, composed of an ureido ring fused with a tetrahydrothiophene ring and a valeric acid substituent attached to one of the carbon atoms of the tetrahydrothiophene ring. Biotin is linked to the rest of the molecule by its terminal carboxylic group through the formation of an amide bond with an adjacent NH group.

Within the meaning of the present invention, for substitution degree or degree of substitution (DS) is intended the number of substituent groups attached per disaccharide unit, expressed as percentage of substituent groups with respect to the total number of disaccharide units. In the present case, the substituent group is selected from the group consisting of biotin, R₄-biotin, R₅-biotin and R₆-biotin. The substitution degree of a compound can be measured according to the general knowledge in the field.

Within the meaning of the present invention, for acetyl group it is intended a COCH₃ group.

Within the meaning of the present invention, the term "alkylene" refers to a divalent alkyl group having two points of attachment within the compound of the invention.

Within the meaning of the present invention, the term "substituted" means that an atom (for example an hydrogen) or an organic group as defined above (e.g., an alkyl group) is replaced by another atom or another organic group.

Within the meaning of the present invention, the term "substituent group" means an organic group that replaces an atom or an organic group of the molecule. According to the present invention, the substituent group is selected from the group consisting of biotin, R₄-biotin, R₅-biotin and R₆-biotin.

According to the present invention, the compound of formula (I) comprises from 1 to 50% substituent groups with respect to the total number of disaccharide units.

It is intended that in a compound of formula (I) not all the units marked with n comprise the same substituent groups in each position R₁, R₁' and R₂.

For example, it is intended that within the same compound of formula (I) in each unit marked with n, R₁ and R₁' can be both OH, or one is OH and the other one is R₄-biotin or they can be both R₄-biotin. Therefore, a compound of formula (I) can comprise some n units wherein both R₁ and R₁' are OH, some n units wherein R₁ is OH and R₁' is R₄-biotin, some n units wherein R₁ is R₄-biotin and R₁' is OH and some n units wherein both R₁ and R₁' are R₄-biotin.

In particular, the substitution degree for the groups R₁ and R₁' is preferably comprised between 1 and 50% of disaccharide units, more preferably between 3 and 30%. More in particular, when both R₁ and R₁' are substituted the substitution degree is preferably comprised between 5 and 15%.

It is also intended that within the same molecule of formula (I) in each unit marked with n, R₂ can be acetyl or it can be substituted with biotin or R₅-biotin. Therefore, a compound of formula (I) can comprise some n units wherein R₂ is an acetyl group and some n units wherein R₂ is biotin or R₅-biotin. Furthermore, in each unit marked with n wherein R₂ is biotin or R₅-biotin, R₁ and R₁' can independently be both OH or they can be both substituted or one can be OH and the other one substituted. As explained above, it is intended that in the same compound of formula (I), not all the units marked with n have the same meanings of R₁, R₁' and R₂. For example, the same compound can comprise some n units not substituted at the R₁ and/or R₁' and substituted at the R₂ and some n units substituted at R₁ and/or R₁' and R₂ and some n units substituted at R₁ and/or R₁' and not at R₂.

It is also intended that in a compound of formula (I) not all the units marked with m comprise the same substituent groups.

For example, it is intended that within the same molecule of formula (I) in each unit marked with m, R₂' can be an acetyl group or it can be substituted by biotin or R₅-biotin. Therefore, the same compound of formula (I) can comprise some m units wherein R₂ is acetyl and some m units wherein R₂ is biotin or R₅-biotin, independently from the meanings of the other R groups.

The substitution degree for the groups R₂ and R₂' is preferably comprised between 1 and 20% of disaccharide units, more preferably between 2 and 10%.

The R₃ group is present on the reducing terminus unit of the compound of formula (I). in some embodiments of the invention R₃ is OH. In other embodiments of the invention, R₃ is a modified disaccharidic moiety comprising a R₆-biotin group. When R₃ is a modified disaccharidic moiety comprising a R₆-biotin group, it has the formula (II) reported above.

In an embodiment, R₃ has the following formula (IIa) wherein U is a glycol-split uronic acid:

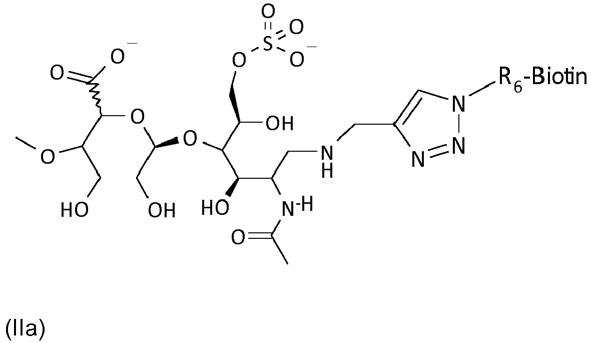

In another embodiment, R₃ has the following formula (IIb) wherein U is 20-sulfated uronic acid:

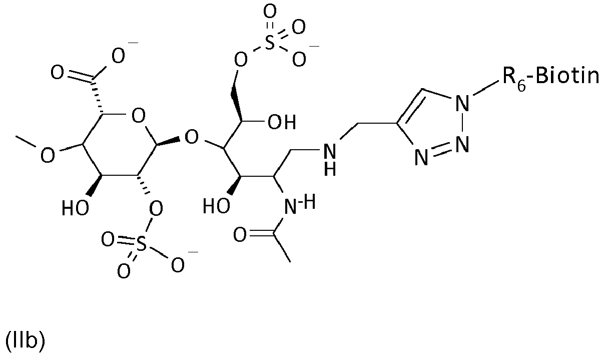

When R₃ is a modified disaccharidic moiety comprising a R₆-biotin group, the "masked aldehyde" at the reducing end of the compound of formula (I) is linked with the amino substituent group.

The substitution degree for the group R₃ is preferably comprised between 1 and 5% of disaccharide units.

Within the meaning of the present invention, C₁-C₆₀alkylene means an alkylene with from one to sixty carbon atoms, wherein each CH₂ group can be optionally substituted by O, NH or CO. Preferably, said alkylene group comprises from 5 to 30 carbon atoms. For example it can comprise one, two, three, four carbon atoms. For example, it can be or comprise a methylene, ethylene, propylene, propylene, butylene, pentylene.

Each of R₄, R₅ and R₆ can have any of the meanings above defined.

In some embodiments, any of said R₄, R₅ and R₆ can comprise an alkylene chain wherein one or more CH₂ group is substituted by one or more of the following groups: O, NH and CO. For example, one or more CH₂ groups in the alkylene chain can be substituted with an oxygen thus forming a polyethylene glycol (PEG). Polyethylene glycol (PEG) is a polyether compound. In the compound of formula (I) the structure of PEG is commonly expressed as -(O-CH₂-CH₂)ₚ-O- with p being preferably comprised from 1 to 10, more preferably from 3 to 4.

In another example, one or more CH₂ groups in the alkylene chain can be substituted with a NH group. In a particular embodiment, any of said R₄, R₅ and R₆ can comprise a cadaverine residue. A cadaverine residue is of the known diamine with the formula NH₂(CH₂)₅NH₂.

In another particular embodiment, any of said R₄, R₅ and R₆ can comprise a pentylamine residue, which is of the known chemical compound with the formula CH₃(CH₂)₄NH₂.

In further embodiments, any of said R₄, R₅ and R₆ can comprise an alkylene chain wherein a hydrogen of one or more CH₂ group is substituted by the side chain of a D or L amino acid

When the side chain of a D,L amino acid is present, it can be from any amino acid, natural or not natural. For example it can be from an amino acid selected from the group consisting of: alanine (Ala), arginine (Arg), asparagine (Asn), aspartic acid (Asp), cysteine (Cys), glutamic acid (Glu), glutamine (Gin), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr) and valine (Val). Side chains of these amino acids are commonly known in the field.

Said R₄, R₅ and R₆ can also comprise an amino acid chain comprising any combination of the above amino acids. In an embodiment, it is an amino acid chain comprising Gly-Gly-Gly. In an embodiment, it is an amino acid chain comprising Ser-Gly-Gly-Gly, preferably repeated for n₂ times, wherein n₂ is comprised between 2 and 10. In an embodiment, it is Gly-Gly-Gly-Ser-Gly-Gly-Gly-Ser-Gly-Gly-Gly.

When R₃ is a modified disaccharidic moiety containing a R₆-biotin group a 1,2,3-triazole group is present at the terminal end. Said group is a known five-membered ring of two carbon atoms and three nitrogen atoms. When 1,2,3-triazole is present in the compound of formula (I) it is 1,4-disubstituted, this meaning that it is linked to the rest of the molecule in positions 1 and 4 on the ring.

In some embodiments, any of R₄, R₅ and R₆ groups comprises one or more carbonyl groups (CO) and/or one or more amine groups (NH).

For example they can comprise an amino group followed by an alkylene chain or a pentylamine residue followed by a carbonyl group or a cadaverine residue followed by a PEG chain.

In an embodiment, the compound of formula (I) comprises at least one group R₄-biotin as R₁ and/or R₁'. In this embodiment, the biotin can be linked to the heparin moiety through an amino group (NH) or a hydrazine group (NHNH), as shown in below scheme 1.

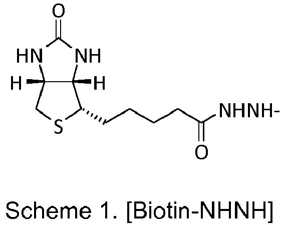

In a preferred embodiment R₄ is or comprises a cadaverine residue with the formula NH(CH₂)₅NH, as shown in following scheme 2.

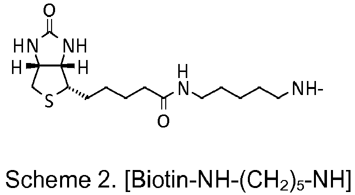

In an exemplary embodiment, R₄-Biotin comprises a PEG residue and a cadaverine residue, as exemplary shown in below scheme 3.

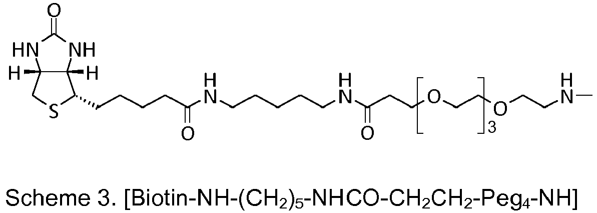

In another embodiment, R₄ comprises an amino acid chain comprising two or more amino acids. When the R₄ group comprises one or more amino acids as groups linking the heparin moiety the terminal amino acid is linked to the glycol split unit through its amino group.

In an embodiment, R₄ comprises an amino acid chain and a cadaverine residue, as shown in below scheme 4.

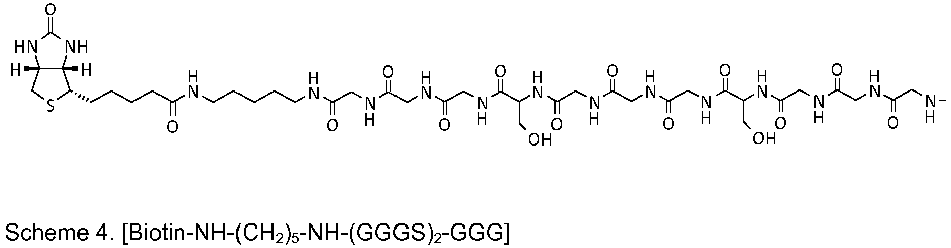

In another embodiment, R₄ comprises a cadaverine residue wherein the terminal amino group is modified in an hydrazide, as shown in below scheme 5.

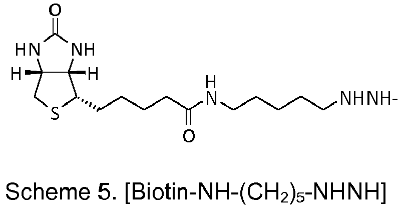

When R₄ is or comprises an amino acid chain, such chain preferably comprises the amino acids Gly and Ser in any combination. In a preferred embodiment, R₄ is or comprises the amino acid chain Gly-Gly-Gly-Ser-Gly-Gly-Gly-Ser-Gly-Gly-Gly.

In an embodiment, the compound of formula (I) comprises at least one group R₅-biotin as R₂ and/or R₂'.

The R₅ group starts with a CO group, facilitating its linkage to the heparin moiety.

In some embodiments, R₅ is absent and the biotin molecule is directly linked to the glucosamine unit through its carbonyl group.

In a preferred embodiment, R₅ is or comprises a pentylamine residue with its amine group linked to the carbonyl of the biotin moiety. In an exemplary embodiment, R₅ comprises a pentylamine residue, as shown in below scheme 6.

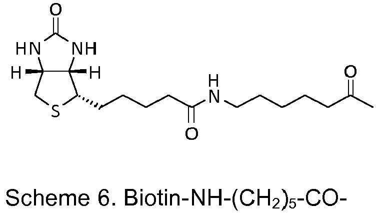

In another embodiment, R₅ comprises a PEG residue and a pentylamine residue, as shown in below scheme 7.

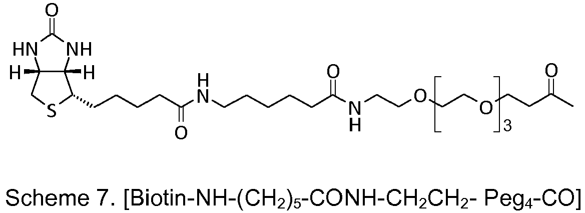

In another embodiment, R₅ comprises an amino acid chain comprising two or more amino acids. When the R₅ group comprises one or more amino acids as groups linking the heparin moiety the terminal amino acid is linked to the glucosamine unit through its carboxyl group.

In an embodiment, R₅ comprises an amino acid chain comprising two or more amino acids and a terminal pentylamine residue.

In an embodiment, the compound of formula (I) comprises at the R₃ position a modified disaccharidic moiety bearing a group R₆-biotin.

R₆ comprises a NH group linked to the terminal carboxylic group of the biotin.

In some embodiments, R₆ can be an amino group (NH) or a -NHNH- group.

In a preferred embodiment R₆ comprises a cadaverine residue with the formula-NH(CH₂)₅NH-.

In other preferred embodiments, R₆ comprises a pentylamine residue with its amine group linked to the biotin moiety.

In another embodiment, R₆ comprises an amino acid or an amino acid chain comprising two or more amino acids. When the R₆ group comprises one or more amino acids as groups linking the heparin moiety the terminal amino acid is linked to the triazole group through its carbon atom previously linked to the free aminogroup of the amino acid.

In an embodiment, R₆ comprises an amino acid or an amino acid chain and a cadaverine residue, as exemplary shown in above scheme 4.

The above described and shown linkers are just exemplary embodiments of the linker of formula (I). Any linker group falling within the definition of formula (I) and not explicitly herein mentioned is within the scope of the invention.

The suitable R_{4/5/6} group can be chosen by the skilled person according to the desired final compound. In particular, the different length and solubility of the groups confer different properties to the final molecule obtained.

For example, the commercial amino-biotin [Biotin-NH₂] is the simplest reagent, having the shortest possible spacer arm; the linker amino-Peg-Biotin, [Biotin-Peg-NH₂] contains a long-chain, albeit simple hydrocarbon, spacer arm that reduces steric hindrance in biotin-binding assays. The group amino-Peg₃-biotin contains a long-chain, water-soluble (hydrophilic), polyethylene glycol (PEG) spacer arm, whose properties are transferred to the final compound.

The skilled person knows how to chose the suitable linker according to the desired properties and the common general knowledge in the field.

In the compound of the invention, n and m preferably vary from 1 to 30 and the sum of n+m preferably ranges from 6 to 30.

Any nitrogen group can be protected. The protective group is preferably selected from tert-butoxycarbonyl, benzyloxycarbonyl and 9-fluorenylmethyloxycarbonyl.

When one or more chiral centers are present in the compounds of the present invention, the individual isomers, enantiomers and diastereoisomers, and mixtures thereof (e.g., racemates) are intended to be encompassed by the present invention.

The pharmaceutical acceptable salts of the compound of formula (I) are included in the scope of the invention.

Pharmaceutical acceptable salts are salts which retain the biological activity of the compound and are derived from known pharmacologically acceptable basis. Examples of pharmaceutically acceptable salts are, for example, those from compounds of formula (I) and inorganic bases, such as sodium, potassium, calcium and aluminum hydroxides, as well as those with organic bases such as, for example, lysine, arginine, N-methyl-glucamine, trimethylamine triethanolamine, dibenzylamine, methylbenzylamine, di-(2-ethyl-hexyl)-amine, piperidine, N-ethylpiperidine, N,N-di-ethylaminoethylamine, N-ethylmorpholine, 3-phenethylamine, N-benzyl-3-phenethylamine, N-benzyl-N,N-dimethylamine. A preferred salt is sodium salt.

Also, pharmaceutically acceptable hydrates or solvates of the compound of formula (I) are included in the scope of the present invention. In particular, they can be any hydrate or solvate commonly used in the art.

Pharmaceutically acceptable salts and their preparation are within the common knowledge of the skilled person and general reference is made to the relevant literature, such as, for example Pharmaceutical Salts and Co-crystals, Johan Wouters, Luc Quéré, Royal Society of Chemistry, 2011.

Preferred compounds according to the present invention are the followings:
N-desulfated N-acetylated glycol split heparin -CO-(PEG)₄-CH₂CH₂-NHCO-(CH₂)₅-NH-Biotin with a substitution degree on the R₂ and/or R₂' positions of about 7% (SST0763NA1), which is a N-desulfated N-acetylated glycol split heparin substituted on the position R₂ and/or on the position R₂' with the group -CO-(PEG)₄-CH₂CH₂-NHCO-(CH₂)₅-NH-Biotin with a substitution degree of about 7%;
N-desulfated N-acetylated glycol split heparin -(GGGS)₃-NH-(CH₂)₅-NH-Biotin with a substitution degree on the R₁ and/or R₁' positions of about 7% (SST0761NA1), which is a N-desulfated N-acetylated glycol split heparin substituted on the position R₁ and/or on the position R₁' with the group -(GGGS)₃-NH-(CH₂)₅-NH-Biotin with a substitution degree of about 7%;
N-desulfated N-acetylated glycol split heparin -Reducing end-NH-CH₂-1,2,3-Triazole-4, N-Glycine-CONH-(CH₂)₅-NH-Biotin with a substitution degree on the R₃ position of about 2 % (SST0764NA1), which is a N-desulfated N-acetylated glycol split heparin substituted on the position R₃ with a modified disaccharidic moiety of formula (II) wherein R₆ is Glycine-CONH-(CH₂)₅-NH- with a substitution degree of about 2%.

Further compounds according to the present invention are the followings:
N-desulfated N-acetylated glycol split heparin -(GGGS)₃-NH-(CH₂)₅-NH-Biotin with a substitution degree on the R₁ and/or R₁' positions of about 3%, and preferably an average molecular weight (MW) of about 14000 Da;
N-desulfated N-acetylated glycol split heparin -NH-(CH₂CH₂O)₄-CH₂CH₂-CONH-(CH₂)₅-NH-Biotin with a substitution degree on the R₁ and/or R₁' positions of about 7%, and preferably an average molecular weight (MW) of about 9500 Da;
N-desulfated N-acetylated glycol split heparin -NH-(CH₂)₅-NH-Biotin with a substitution degree on the R₁ and/or R₁' positions of about 31%, and preferably an average molecular weight (MW) of about 15500 Da;
N-desulfated N-acetylated glycol split heparin -NH-(CH₂)₅-NH-Biotin with a substitution degree on the R₁ and/or R₁' positions of about 12%, and preferably an average molecular weight (MW) of about 16000 Da;
N-desulfated N-acetylated glycol split heparin -NH-(CH₂)₅-NH-Biotin with a substitution degree on the R₁ and/or R₁' positions of about 41%, and preferably an average molecular weight (MW) of about 17000 Da;
N-desulfated N-acetylated glycol split heparin -NH-(PEG)₄-CH₂CH₂-CONH-(CH₂)₅-NH-Biotin with a substitution degree on the R₁ and/or R₁' positions of about 21%, and preferably an average molecular weight (MW) of about 18500 Da;
N-desulfated N-acetylated glycol split heparin -NH-(PEG)₄-CH₂CH₂-CONH-(CH₂)₅-NH-Biotin with a substitution degree on the R₁ and/or R₁' positions of about 28%, and preferably an average molecular weight (MW) of about 21000 Da;
N-desulfated N-acetylated glycol split heparin -CO-(CH₂)₅-NH-Biotin with a substitution degree on the R₂ and/or R₂' positions of about 15%, and preferably an average molecular weight (MW) of about 9500 Da;
N-desulfated N-acetylated glycol split heparin -CO-(PEG)₄-CH₂CH₂-NHCO-(CH₂)₅-NH-Biotin with a substitution degree on the R₂ and/or R₂' positions of about 7%, and preferably an average molecular weight (MW) of about 8500 Da;
N-desulfated N-acetylated glycol split heparin -Reducing end-NH-CH₂-1,2,3-Triazole-4, N-(CH₂)₅-NH-Biotin with a substitution degree on the R₃ position of about 4%, and preferably an average molecular weight (MW) of about 9500 Da;
N-desulfated N-acetylated glycol split heparin -Reducing end-NH-CH₂-1,2,3-Triazole-4, N-Glycine-CONH-(CH₂)₅-NH-Biotin with a substitution degree on the R₃ position of about 2%, and preferably an average molecular weight (MW) of about 9000 Da;
wherein "Reducing end" means that the linker is on the reducing end of the molecule.

### Synthesis of the linker groups

The R_{4/5/6}-biotin groups, also herein named as "linkers", can be synthesized by many methods available to those skilled in the art of organic chemistry. Such linkers are synthetized in an activated form allowing their linkage to the heparin moiety.

General and exemplary synthetic schemes for preparing said linkers are described below. These schemes are illustrative and are not meant to limit the possible techniques one skilled in the art may use to prepare the linkers disclosed herein. Different methods to prepare the compounds of the present invention will be evident to those skilled in the art. Additionally, the various steps in the synthesis may be performed in an alternate sequence in order to give the desired linker or linkers.

Also, the skilled in the art can easily alter the reagents and reaction conditions exemplified in the schemes below to include any combination of groups encompassed by the present invention. Also, the skilled artisan can easily use interchangeable steps for each synthetic process and incorporate isolation and/or purification steps as deemed necessary.

All the conditions and reagents not explicitly mentioned below can be easily chosen by the skilled in the art according to the general knowledge in the field.

Many R_{4/5/6}-biotin groups according to the present invention are already commercially available or they can be obtained from commercial compounds by well-known synthesis methods.

Exemplary procedures for the obtainments of R_{4/5/6}-biotin linkers are described and shown below.

### Linkers on the glycol split (R₄-biotin linkers)

For the synthesis of some exemplary R₄-biotin groups the following procedures can be used.

R₄-biotin linkers wherein R₄ is an amino group (NH) or two amino groups (NHNH) are commercially available.

R₄-biotin linkers wherein R₄ is a cadaverine group are commercially available, for example as 5-(Biotinamido)pentylamine.

R₄-biotin linkers wherein R₄ is a cadaverine group followed by an amine group are commercially available.

R₄-biotin linkers wherein R₄ comprises a cadaverine group can be obtained starting from commercially available 5-(Biotinamido)pentylamine and adding the desired linker portion.

For example, R₄-biotin linkers wherein R₄ comprises a cadaverine residue and a PEG group can be obtained as represented in the following scheme 8.

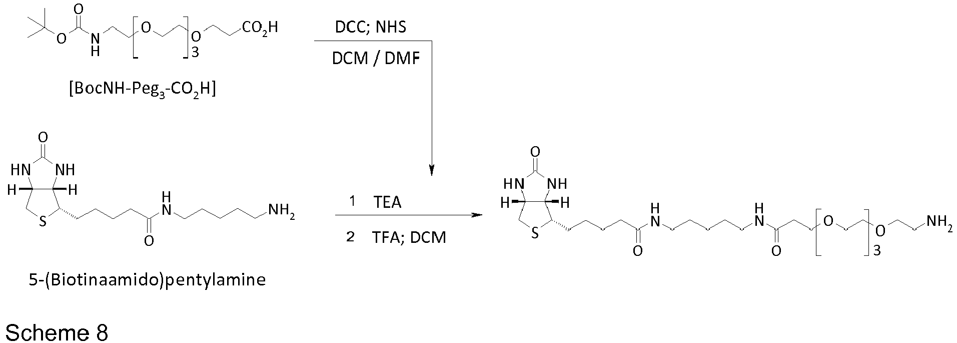

As represented in above scheme 8, the commercial product, 15-(t-Butyloxycarbonyl)amino-4,7,10,13-teraoxa-pentadecanoic acid (Boc-NHPeg₄-CC2H) is dissolved in Dichloromethane (DCM) and cooled, for example to 0°C. Suitable reagents such as N-hydroxy succinimide (NHS) and Dicyclohexylcarbodiimide (DCC) are added to the reaction mixture and stirred at low temperature, for example 0°C. Next, to the activated solution, 5-(Biotinamido)pentylamine, commercially available, dissolved in a suitable solvent, such as Dimethylformamide (DMF) and Triethylamine (TEA), is added thus obtaining Biotin-cadaverine-CO-(CH₂)₂Peg-NH-Boc, which is then deprotected with conventional technique, for example with Trifluoroacetic acid (TFA), to obtain Biotin- cadaverine-CO-(CH₂)₂Peg-NH₂.

Groups with different lengths of the Peg chain can be obtained by repeating the same process.

R₄-biotin linkers wherein R₄ comprises a cadaverine residue and an amino acid chain comprising two or more amino acids can be obtained as described below.

An exemplary process is shown in the following Scheme 9.

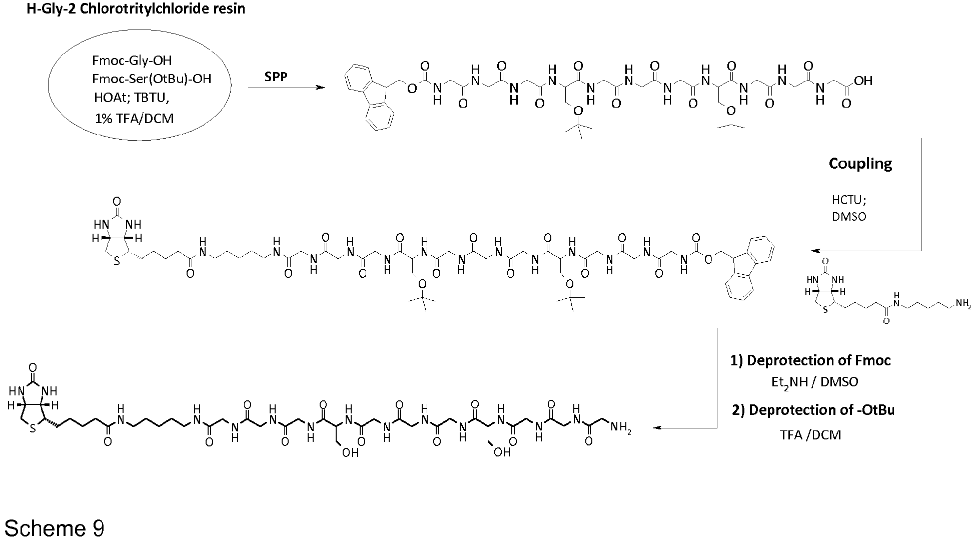

As represented in above scheme 9, FmocNH-GGG-[S(OtBu)GGG]₂-OH is first obtained by chemical synthesis using Fmoc-chemistry on solid phase. The general principle of this known method is based on repeated coupling and deprotection cycles using the 9H-fluoren-9-ylmethoxycarbonyl, as a protective group of the N-terminal end of growing peptides. Each Fmoc-Amino acid is coupled by coupling reagent, such as 1-Hydroxybenzotriazole (HOBt) and *N,N,N',N'* -Tetramethyl-*O*-(benzotriazol-1-yl)uronium tetrafluoroborate (TBTU) and *N-*Ethyldiisopropylamine (DIEA), in suitable solvents, such as Dimethylformamide (DMF), for example with Microwave-heating assisted irradiated at 30 W for 5 min. The Fmoc-group is then removed using for example 20% piperidine in DMF under MW irradiation. Each coupling and deprotection is usually conducted only once; a second coupling cycle may be needed, for example for the second Fmoc-Ser(OtBu)-OH. Following completion of the sequence, the linear peptide (for example of eleven amino acids) is cleaved from resin for example by treatment with 1% TFA in CH₂Cl₂. The desired amino acid sequence, for example in the scheme above Fmoc-NH-GGG[S(OtBu)GGG]₂-OH, is thus obtained.

The linker with biotin is obtained by activating FmocNH-GGG-(S(OtBu)GGG)₂-OH with suitable reagents such as O-(6-Chlorobenzotriazol-1-yl)-N,N,N',N'-tetramethyluronium hexafluorophosphate (HCTU) and DIEA. The activated solution is then added to commercial compound 5-(Biotinamido)-pentylamine. To FmocNH-GGG-[(S(OtBu)GGG)]₂-cadaverine-Biotin, Diethylamine (Et₂NH) is added until total removal of Fmoc-group. Deprotection is finally performed, for example using TFA, to obtain the final group NH₂-GGG-(SGGG)₂-cadaverine-Biotin.

Different groups with other amino acids or amino acid chains can be obtained with the same general procedure above described by using the suitable Fmoc-amino acids.

### Linkers on the glucosamine portion (R₅-biotin linkers)

For the synthesis of some exemplary R₅-biotin groups the following exemplary procedures can be used.

In some embodiments, R₅ is absent and the biotin molecule is directly linked to the glucosamine unit through its carbonyl group. In this case, to the commercial product 6-Biotinylamino-hexanoic acid (Biotin-Ahx-OH) N-hydroxysuccinimide and N-(3-Dimethylaminopropyl)-N' ethyl-carbodiimide are added to give activated Biotin-OSu.

The N-Hydroxysuccinimide ester (NHS) terminal group is commonly used to covalently couple amine-containing molecules via amide linkages.

These linkers are synthetized in an activated form allowing their linkage to the heparin portion.

Linkers wherein R₅ is or comprises pentylamine can be obtained by activation of the commercial product 6-Biotinylamino-hexanoic Acid (Biotin-Ahx-OH) by suitable reagents such as N-hydroxysuccinimide (NHS) and N-(3-Dimethylaminopropyl)-N' ethyl-carbodiimide (EDC) as exemplary shown in below scheme 10b.

Linkers wherein R₅ comprises a pentylamine can be obtained by adding the desired linker groups to the above mentioned CO-pentylamine-biotin compound.

In an exemplary embodiment, a linker comprising a pentylamine residue and a PEG group can be obtained as described below. An exemplary process is shown in the following Scheme 10a.

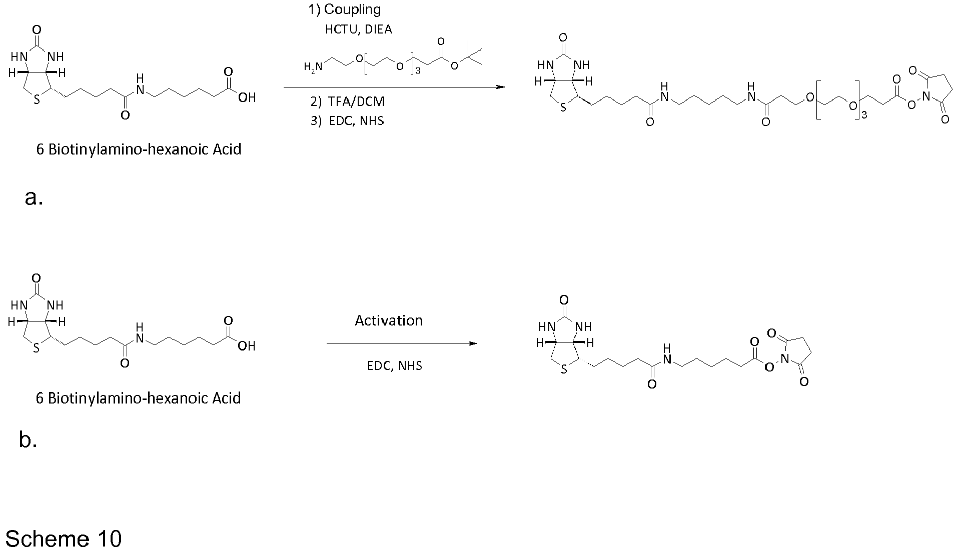

The commercial product 6-Biotinylamino-hexanoic Acid (Biotin-Ahx-OH) is activated by suitable reagents such as HCTU and DIEA. The activated solution is then added to commercial compound tBuO-Peg₃-NH₂ thus obtaining Biotin-Peg₃-OtBu. Then, other DIEA can be added. The obtained compound Biotin-Peg₃-OtBu is deprotected, for example with trifluoroacetic acid (TFA). To obtained Biotin-Peg₃-OH, N-hydroxy succinimide and N-(3-Dimethylaminopropyl)-N' ethylcarbodiimide are added to obtain Biotin-Peg₃-OSu activated (scheme 10a).

Different lengths of the PEG chain can be obtained by adding the suitable compound to the activated Biotin-Ahx-OH.

### Linkers on the terminal reducing end (R₆-biotin linkers)

For the synthesis of some exemplary R₆-biotin groups the following exemplary procedures can be used.

Linkers wherein R₆ is an amino group (NH) or two amino groups (NHNH) or comprises a cadaverine group are commercially available or can be obtained by procedures above explained for R₄-biotin linker.

Exemplary linkers wherein R₆ comprises a cadaverine residue or a pentylamine residue can be obtained as shown in following scheme 11.

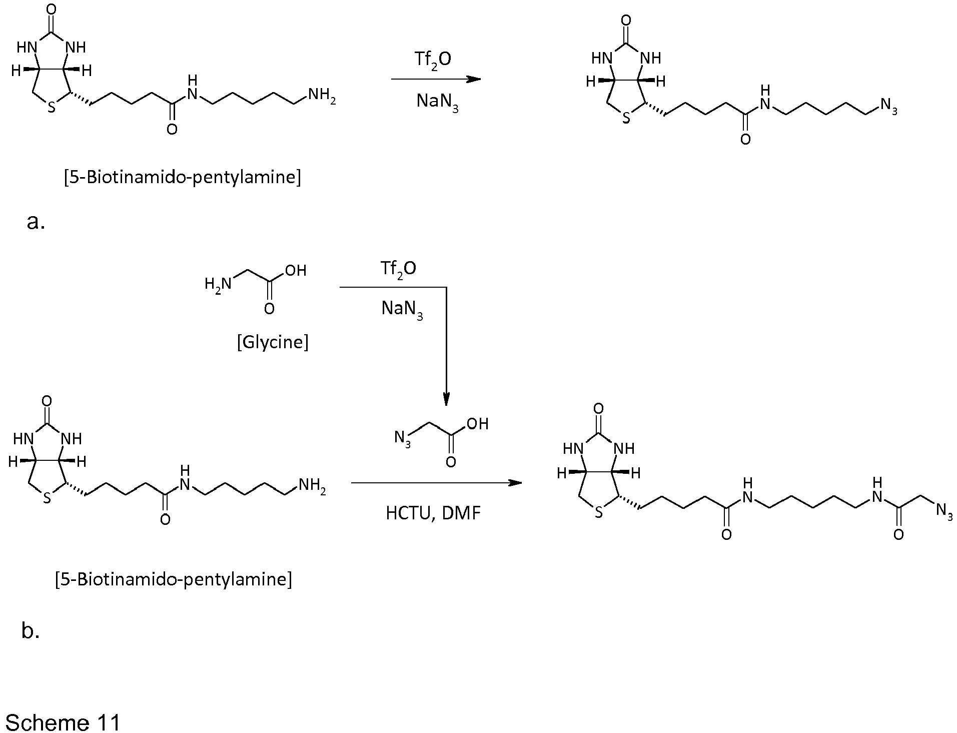

To obtain linkers wherein R₆ comprises a pentylamine group, the following procedure can be used. To prepare TfN₃, first NaN₃ is added to CH₂Cl₂; Tf₂O is then added. Commercial Biotine cadaverine (5-Biotinamido-pentyamine), is then added to the obtained solution of TfN₃, thus obtaining biotine-pentylammine-N₃, as represented in above scheme 11 a.

To obtain linkers wherein R₆ comprises a cadaverine group, the following procedure can be used.

A solution of NaN₃ is treated with CH₂Cl₂. The resulting biphasic mixture is treated with Tf₂O. Commercial compound Glycine is added to obtained TfN₃, thus obtaining N₃-Gly-OH. To N₃-Gly-OH, suitable reagents such as HCTU and DIEA are added. The solution of Gly-Azide is added to a solution of commercial compound Biotine-cadaverine ((5-Biotinamido)-pentyamine) to obtain the linker biotin-cadaverine-glycine-azide, as shown in above scheme 11 b.

Any other amino acid or amino acid chain can be introduced in such groups by adding the corresponding commercial compound to obtained TfN₃.

Typically, such obtained linkers are used in a click chemistry reaction, as explained below, giving rise to a 1,2,3-triazole-1,4-disubstituted group.

Any other linker falling within the scope of formula (I) is commercially available or can be easily obtainable by the skilled person according to the general knowledge and practice in the field, for example starting from the processes above disclosed.

### Synthesis processes of the compounds of the invention

The compounds of the present invention can be synthesized by many methods available to those skilled in the art of organic chemistry. General and exemplary synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared by methods described in the general schemes are given in the intermediates and examples section set out hereinafter.

The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed.

It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene et al. (Protective Groups in Organic Synthesis, 4th Edition, Wiley- Interscience (2006)).

Also, the skilled in the art can easily alter the reagents and reaction conditions exemplified in the schemes below to include any combination of substituents encompassed by the present invention. Also, the skilled artisan can easily use interchangeable steps for each synthetic process and incorporate isolation and/or purification steps as deemed necessary.

All the conditions and reagents not explicitly mentioned below can be easily chosen by the skilled in the art according to the general knowledge in the field.

Starting materials useful for the preparing the compounds of the invention and intermediates therefor are commercially available or can be prepared by well known synthetic methods.

Exemplary processes for the synthesis of the compounds of the invention are herein described.

Compounds of the invention can be obtained starting from known N-desulphated heparin using the known reaction steps for preparation of N-acetylated-glycol split-heparin in the appropriate sequence, in function of the substitution position, and adding the desired group biotin, R₄-biotin, R₅-biotin or R₆-biotin, as will be exemplary described in the following.

Known N-desulphated N-acetylated-glycol split-heparin can be obtained for example starting from heparin, which is N-desulphated, for example in a mixture of DMSO in water 95:5 to obtain a desulphation degree of about 100%, and then N-acetylated, for example using acetic anhydride. The obtained N-acetylated heparin is oxidized, for example using sodium periodate, obtaining a N-acetylated-glycol split-heparin-aldehyde, to which suitable substituent groups according to the present invention can be added. For the obtainment of N-desulphated N-acetylated-glycol split-heparin reference can be made, for example to WO03022291 and EP2343077.

In an embodiment, a compound of formula (I) comprising at least one group R₄-biotin as R₁ and/or R₁' is obtained by a process comprising the following steps:
a) N-desulphation of heparin, for example in a mixture of Dimethyl sulfoxide (DMSO) in water 95:5, to obtain a desulphation degree of about 100%;
b) N-acetylation of the obtained N-desulphated heparin, for example using acetic anhydride;
c) oxidation of the obtained N-acetylated heparin, for example using sodium periodate, thus obtaining a N-acetylated-glycol split-heparin-aldehyde;
d) addition of the R₄-biotin group;
e) reduction, for example using a borohydride, for example sodium cyanoborohydride;
f) optionally, further reduction, for example using a borohydride such as sodium borohydride.

An exemplary process for the preparation of compounds of formula (I) comprising at least one group R₄-biotin as R₁ and/or R₁' is herein described.

An exemplary process is shown in following scheme 12.

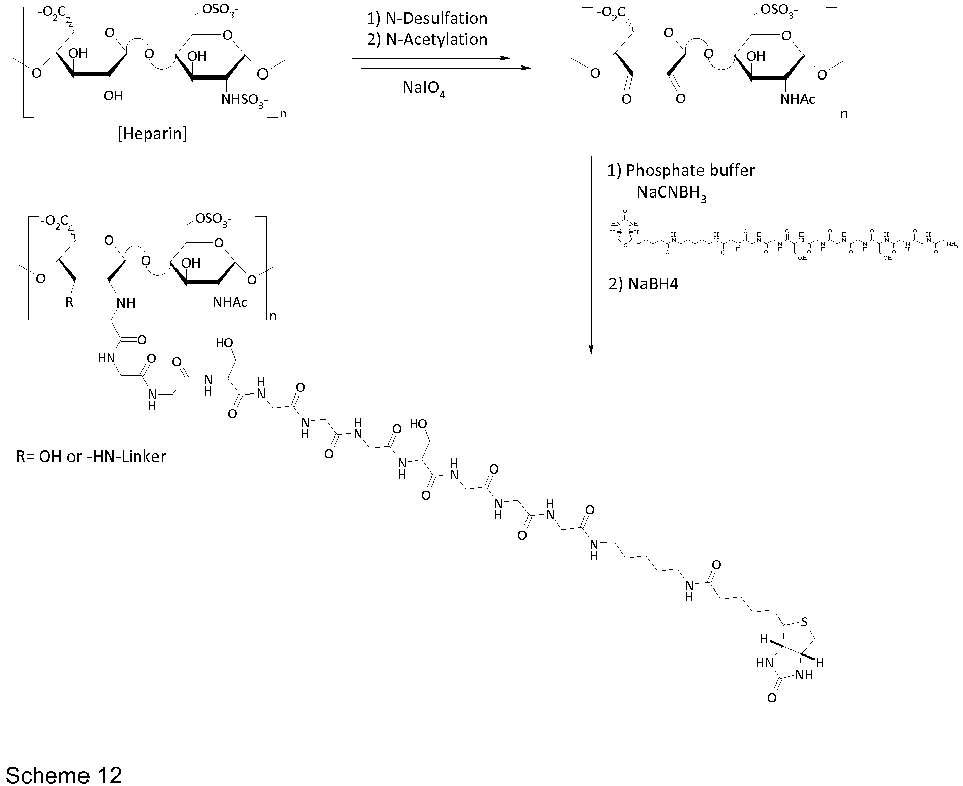

Heparin is N-desulphated, for example in a mixture of DMSO in water 95:5 to obtain a desulphation degree of about 100%, and then N-acetylated, for example using acetic anhydride. The obtained N-acetylated heparin is oxidized, for example using sodium periodate, obtaining a N-acetylated-glycol split-heparin-aldehyde. For these synthesis steps, reference can also be made to WO03022291. The suitable R₄-biotin group is then added. Reduction is then performed, for example using a borohydride, for example sodium cyanoborohydride. Finally, a further reduction can be performed, for example using a borohydride such as sodium borohydride. The desired biotinylated heparin with the desired substitution degree is thus obtained. The skilled person in the field knows how to obtain the desired substitution degree according to his general knowledge in the field. The substitution degree of the final compound can be measured according to the general knowledge in the field.

An exemplary R₄-biotin group is shown in scheme 12 above but the process is equally applicable to any R₄-biotin group according to the invention.

In an embodiment, a compound of formula (I) comprising at least one group biotin or R₅-biotin as R₂ and/or R₂' is obtained by a process comprising the following steps:
a) N-desulphation of heparin, for example in a mixture of DMSO in water 95:5, to obtain a desulphation degree of about 100%;
b) oxidation-reduction reaction of the obtained N-desulphated heparin, for example using sodium periodate, obtaining a N-desulphated-glycol split-heparin;
c) addition of the biotin or R₅-biotin group;
d) N-acetylation, for example using acetic anhydride.

An exemplary process for the preparation of compounds of formula (I) comprising at least one group R₅-biotin as R₂ and/or R₂' is herein described and shown in following scheme 13.

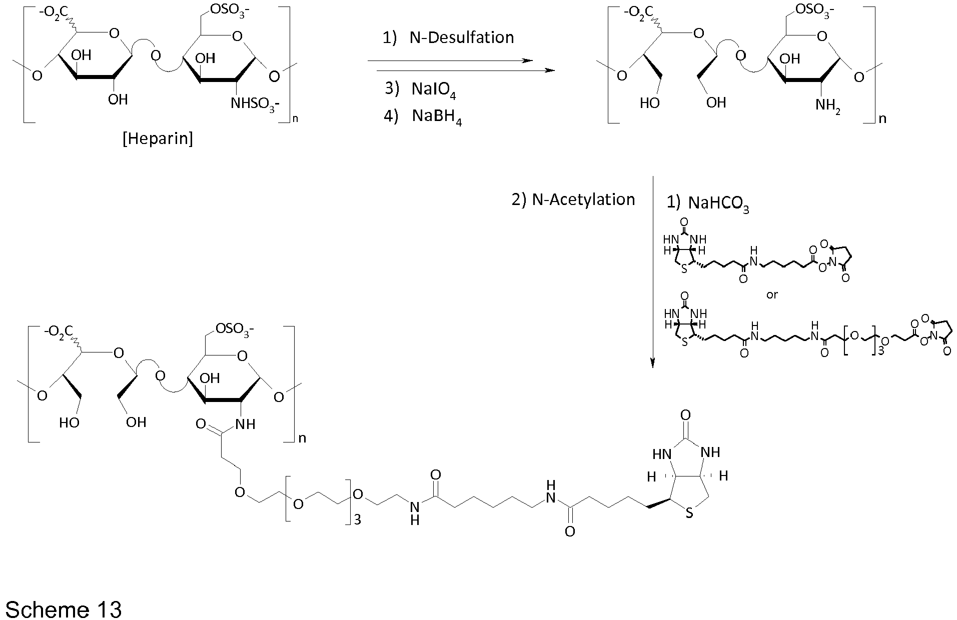

Heparin is N-desulphated, for example in a mixture of DMSO in water 95:5 to obtain a desulphation degree of about 100%, and then a redox reaction is carried out using for example sodium periodate. The N-desulphated-glycol split-heparin is then conjugated with a properly activated R₅-biotin group and finally acetylated, for example by adding acetic anhydride. In scheme 13 above only the glucosamine linked to the glycol-split unit is shown, however the mentioned process of conjugation and acetylation can be equally applied to the glucosamine linked to the sulfated uronic acid.

The skilled person in the field knows how to obtain a compound with the desired substitution degree on the glucosamine unit according to his general knowledge in the field.

In scheme 13, an exemplary process with exemplary R₅-biotin groups is shown. However, the process can be equally carried out with any biotin or R₅-biotin group according to the present invention.

In an embodiment, a compound of formula (I) wherein R₃ is a disaccharidic moiety of formula (II) comprising at least one group R₆-biotin is obtained by a process comprising the following steps:
a) N-desulphation of heparin, for example in a mixture of DMSO in water 95:5, to obtain a desulphation degree of about 100%;
b) N-acetylation of the obtained N-desulphated heparin, for example using acetic anhydride;
c) reductive amination, for example using 2-amino propargyl and sodium cyanoborohydride;
d) oxidation-reduction reaction, for example using sodium periodate and sodium borohydride;
e) conjugation with the R₆-biotin group by click-chemistry reaction.

An exemplary process for the preparation of compounds of formula (I) comprising at least one group R₆-biotin in position R₃ is herein described. An exemplary process is shown in following scheme 14.

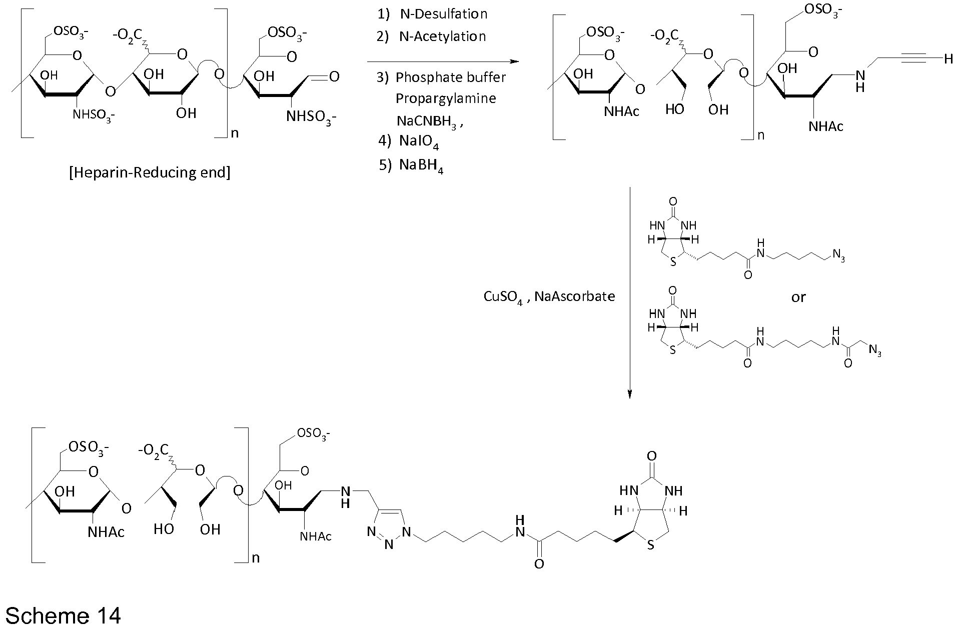

In solution, the reducing end residues of heparin chains are in an equilibrium state between the cyclic and the acyclic form. In the acyclic form the aldehyde can form an imine with one primary amino group of 2 amino propargyl. Because this imine is not stable enough, it is best stabilized by its reduction to a secondary amine using sodium cyanoborohydride.

Heparin is N-desulphated, for example in a mixture of DMSO in water 95:5 to obtain a desulphation degree of about 100%, and then N-acetylated, for example using acetic anhydride, as already above explained. The obtained compound then undergoes reductive amination by adding, for example, 2-amino propargyl and sodium cyanoborohydride. A redox reaction is then carried out using for example sodium periodate and sodium borohydrate. The obtained heparin is then conjugated with a suitable linker-biotin group by click chemistry reaction thus obtaining the desired final compound.

The Click-chemistry approach based on Huisgen's 1,3-dipolar cycloaddition reaction of alkynes to azides selectively gives 1,2,3-triazole derivatives. The click reactions are reactions known in the field. A copper-catalyzed reaction, usually conducted under aqueous condition, at room temperature, allows the synthesis of the 1,4-disubstituted regioisomers specifically. The active Cu(I) catalyst can be generated from Cu(I)salts or Cu(ll) salts using sodium ascorbate as the reducing agent. For this kind of reactions reference can be made to: Kolb, H. C., & Sharpless, K. B. (2003). The growing impact of click chemistry on drug discovery. Drug discovery today, 8(24), 1128-1137.; Tanaka, K., Kageyama, C., & Fukase, K. (2007). Acceleration of Cu (I)-mediated Huisgen 1, 3-dipolar cycloaddition by histidine derivatives. Tetrahedron Letters, 48(37), 6475-6479. Fajardo, A. R., Guerry, A., Britta, E. A., Nakamura, C. V., Muniz, E. C., Borsali, R., & Halila, S. (2014). Sulfated glycosaminoglycan-based block copolymer: preparation of biocompatible chondroitin sulfate-b-poly (lactic acid) micelles. Biomacromolecules, 15(7), 2691-2700.ln scheme 14, an exemplary process with exemplary R₆-biotin groups is shown. However, the process can be equally used with any R₆-biotin group according to the present invention.

All the transformations above described are only exemplary synthetic procedures already known in organic chemistry and well known to the experts in the field.

Any variation or combination of substituents of compounds of formula (I) which is within the scope of the present invention and is not explicitly shown or described in the above processes, can be easily obtained starting from the exemplary processes described above with suitable modifications, for example in the starting compounds or reagents, that are well within the knowledge of the skilled person in the field of organic chemistry. Reference can be made, for example, to the handbook "March's Advanced Organic Chemistry, Reactions, Mechanism and structures", Michael B. Smith, Jerry March, last edition.

### Medical uses

According to the present invention, the compounds of formula (I) are endowed with anti-heparanase activity. Therefore, they can be advantageously used for the treatment of diseases which can be improved or prevented by the inhibition of heparanase activity.

The heparanase has a critical role in modulating the microenvironment via the ECM (extracellular matrix) remodeling and thus influencing a multitude of both normal and disease-related processes, as inflammation, blood coagulation, wound healing, angiogenesis, fibrosis and tumor cell growth, invasion and metastasis (for a review of the roles and activities of heparanase see for example "Heparanase: From basic research to therapeutic applications in cancer and inflammation", Vlodavsky I., Singh P., Boyango I., Gutter-Kapon L., Elkin M., Sanderson RD, Ilan N., Drug Resist Updat. 2016 Nov;29:54-75). Heparanase has also been recently reported to play a fundamental role in many other pathologies, such as nephropathies of different origin, severe infective disorders, gastrointestinal diseases, osteolysis in bone metastases as well as in other bone tissue related pathologies, atherosclerosis, cardiovascular disorders and cutaneous aging. The panorama of diseases affected by heparanase expression and activity also includes the so-called rare diseases, such as amyloidosis, hereditary multiple exostoses and idiopathic avascular necrosis of bone as well as pathologies that are not rare in terms of incidence, but are rarely diagnosed, such as vulvodynia (Rivara et al., 2016).

Therefore, the compounds of formula (I) can be advantageously used in a broad range of diseases.

In particular, heparanase expression is enhanced in cancers, including various carcinomas, sarcomas and hematological malignancies, and it has been demonstrated that it correlates with increased tumor size, tumor angiogenesis, enhanced metastasis and poor prognosis. Indeed, treatments of tumor-bearing mice with heparanase-inhibiting compounds has been shown to markedly attenuate tumor progression further supporting an anti-heparanase therapy for multiple types of cancer (Vlodavski et al., 2016).

Therefore, the compounds of the invention, thanks to their demonstrated activity as heparanase inhibitors, can be advantageously used in the treatment of tumors and metastasis.

In particular, they can be used in the treatment of any kind of tumor, both solid and liquid. For example they can be used in the treatment of solid tumors, such as for example glioma and sarcomas, such as fibrosarcoma and osteosarcoma, and in the treatment of hematological malignancies.

It has been found that the biotinylated compounds of the invention are endowed with anticancer and anti-metastatic activity, comparable to or even higher than the not biotinylated compounds. Therefore, compounds of the invention can be advantageously used for the same medical treatments of known N-desulfated N-acetylated glycol split heparin but with the further advantage of comprising a biotin moiety.

The involvement of heparanase in inflammatory reactions has also been shown (Vlodavski et al., 2016) thus supporting the use of inhibitors of heparanase also in diseases characterized by or related with acute and chronic inflammation, such as colitis, atherosclerosis, pancreatitis, Chron's disease, psoriasis and others.

More recently, heparanase overexpression has been documented to be related to the endothelial mesenchymal transition (EMT; Masola V et al. Specific heparanase inhibition reverses glucose-induced mesothelial-to-mesenchymal transition Nephrol Dial Transplant 2017; 1-9) and thus heparanase inhibition may be also beneficial in those pathologic processes involving EMT, as fibrosis occurring at different organs.

Examples of diseases which can be treated by compounds of formula (I) are tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis (in particular, peritoneal fibrosis glucose induced following peritoneal dialysis) and aging.

The skilled in the art, for example a physician, can identify and recognize if a disease can be ameliorated by an anti-heparanase activity on the basis of the common knowledge in the field. For example, for a broad, even though not exhaustive, overview, on possible clinical applications of heparanase inhibitors, reference can be made to the reviews of Rivara et al., 2016 and Vlodavski et al., 2016. Also, tests are known in the field and available to the skilled person to evaluate if a compound is endowed with an anti-heparanase activity, for example the AGAIA assay (Hammond E. et al. Anal. Biochem. 2010, 396, 112).

Compounds of the invention are particularly advantageous also for non-tumoral applications since they are endowed with a strong anti-heparanase activity and therefore they can already be efficiently used at low doses at which they do not have a significant anti-proliferative effect. Indeed, in some embodiments they are selective heparanase inhibitors already at concentrations in which they do not have a significant anti-proliferative effect. This renders them particularly useful for therapeutic applications wherein a cytotoxic or anti-proliferative effect is undesired. Also, it has been found that such compounds at not cytotoxic concentrations are already able to inhibit invasion of tumor cells thus being particularly useful in the treatment of tumors and metastasis, also as adjuvants of chemotherapeutic agents.

The compounds of the invention are also able to interfere with adhesion properties of cancer cells and they have an inhibitory effect against tumor transcription genes encoding for proangiogenic factors thus further supporting their therapeutic use in cancer diseases and metastasis.

The skilled person in the field knows how to determine the suitable dose in view of the desired application and his general knowledge in the medical and pharmaceutical field and by carrying out routine experimentation.

Another object of the present invention is a pharmaceutical composition containing at least one compound of formula (I) as an active ingredient, in an amount such as to produce a significant therapeutic effect, together with pharmaceutically acceptable vehicles and/or excipients.

Such pharmaceutical compositions can be prepared by conventional methods and techniques which are common practice in the pharmaceutical industry, such as, for example, those illustrated in Remington's Pharmaceutical Science Handbook, Mack Pub. N.Y. - last edition.

The compositions according to the present invention contain, along with the active ingredient, at least one pharmaceutically acceptable vehicle or excipient. These may be particularly useful formulation coadjuvants, e.g. solubilising agents, dispersing agents, suspension agents, and emulsifying agents.

The pharmaceutical compositions according to the invention comprising the compound of formula (I) can also contain one or more further active ingredients, for example chemotherapeutic agents. In particular, when the pharmaceutical composition is used for the treatment of a tumor, it preferably comprises also a chemotherapeutic agent. Indeed in some embodiments, the compounds of the invention act by modifying the tumor microenvironment to affect cell growth and spread as well as to facilitate the tumor killing action of other agents. This makes possible the combination of the compounds of the invention with any chemotherapeutic agent, which can be chosen among the known agents according to the general knowledge of the skilled person in the field. For example, said chemotherapeutic agent can be selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens. When said chemotherapeutic agent is a cytotoxic agent, it is preferably selected from the group consisting of melphalan, irinotecan, rituximab and lapatinib. When said chemotherapeutic agent is a proteasome inhibitor, it is preferably selected from the group consisting of bortezomib, carfilzomib and ixazomib. When said chemotherapeutic agent is an immunomodulatory agent, it is preferably selected from the group consisting of thalidomide, lenalidomide and pomalidomide. For example, when the disease to be treated is multiple myeloma (MM), the compounds of the invention can be administered in conjunction with chemotherapy drugs known to be effective in MM, such as for example cytotoxic agents, for example melphalan, proteasome inhibitors, immunomodulatory drugs (IMIDs) and monoclonal antibodies towards highly expressed myeloma associated antigens.

According to the administration route chosen, the compositions will be in solid or liquid form, suitable for oral, parenteral or topical administration. A preferred administration mode is parenteral administration.

Generally, the compounds of this invention are administered in a "therapeutically effective amount". The amount of the compound actually administered will typically be determined by a physician, in the light of the relevant circumstances, including the condition to be treated, the chosen route of administration, the actual compound administered, drug combination, the age, body weight, and response of the individual patient, the severity of the patient's symptoms, and the like. For any compound, the therapeutically effective dose can be estimated initially either in cell culture assays or in animal models, usually mice, rats, guinea pigs, rabbits, dogs, or pigs. The animal model may also be used to determine the appropriate concentration range and route of administration. Such information can then be used to determine useful doses and routes for administration in humans. In calculating the Human Equivalent Dose (HED) it is recommended to use the conversion table provided in Guidance for Industry and Reviewers document (2002, U.S. Food and Drug Administration, Rockville, Maryland, USA).

Generally, an effective dose will be from 0.01 mg/kg to 100 mg/kg, preferably 0.05 mg/kg to 50 mg/kg. For any compound, the therapeutically effective dose can be estimated initially either in cell culture assays or in animal models, usually mice, rats, guinea pigs, rabbits, dogs, or pigs. The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

Compositions may be administered individually to a patient or may be administered in combination with other agents, drugs or hormones. For example they can be used in combination with further active ingredients, such as for example anticancer drugs.

The medicament may also contain a pharmaceutically acceptable carrier, for administration of a therapeutic agent. Such carriers include antibodies and other polypeptides, genes and other therapeutic agents such as liposomes, provided that the carrier does not induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

A thorough discussion of pharmaceutically acceptable carriers is available in Remington's Pharmaceutical Sciences (Mack Pub. Co., N.J. 1991).

Pharmaceutically acceptable carriers in therapeutic compositions may additionally contain liquids such as water, saline, glycerol and ethanol.

Additionally, auxiliary substances, such as wetting or emulsifying agents, pH buffering substances, and the like, may be present in such compositions. Such carriers may enable the pharmaceutical compositions to be formulated as tablets, pills, dragees, capsules, liquids, gels, syrups, slurries, suspensions, and the like, for ingestion by the patient.

Once formulated, the compositions of the invention can be administered directly to the subject. The subjects to be treated can be animals; in particular, human subjects can be treated.

The medicament of this invention may be administered by any number of routes including, but not limited to, oral, intravenous, intramuscular, intra-arterial, intramedullary, intrathecal, intraventricular, transdermal or transcutaneous applications, subcutaneous, intraperitoneal, intranasal, enteral, topical, sublingual, intravaginal or rectal means.

The compositions for oral administration may take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include refilled, pre-measured ampoules or syringes of the liquid compositions or pills, tablets, capsules or the like in the case of solid compositions. In such compositions, the compound of the invention is usually a minor component (from about 0.1 to about 50% by weight or preferably from about 1 to about 40% by weight) with the remainder being various vehicles or carriers and processing aids helpful for forming the desired dosing form. Dosage treatment may be a single dose schedule or a multiple dose schedule.

A further object of the invention is a process for the preparation of pharmaceutical compositions characterized by mixing one or more compounds of formula (I) with suitable excipients, stabilizers and/or pharmaceutically acceptable diluents.

It is also an object of the invention the use of such pharmaceutical compositions as a medicament, in particular in the treatment of a disease which can be improved or prevented by an anti-heparanase activity. More in particular, the above mentioned diseases can be treated by such pharmaceutical compositions.

Pharmaceutical compositions and preparations thereof are within the general knowledge and reference can be made to conventional manuals and national and European Pharmacopoeias.

In an embodiment, the compounds of the invention can also be used for cosmetic applications thanks to their activity of inhibition of heparanase. Indeed, heparanase is produced in the skin by epidermal keratinocytes composing the epidermis and fibroblasts or vascular endothelial cells of the dermis and it decomposes heparan sulfate proteoglycan, which regulates differentiation and growth of the epidermis. It is known that heparanase can promote the formation of wrinkles; indeed, inhibition of heparanase activity suppresses the release of growth factors that accompanies decomposition of heparan sulfate, and allows migration of growth factors between the epidermis and dermis to be controlled, thereby aiding in anti-aging and anti-wrinkles effects of the skin (see for example EP2484349, US2014080878 and the work "Hyperpigmentation in human solar lentigo is promoted by heparanase-induced loss of heparan sulfate chains at the dermal-epidermal junction." Iriyama S, Ono T, Aoki H, Amano S. Dermatol Sci. 2011 Dec;64(3):223-8).

In view of the above and of the heparanase inhibition activity of the compounds of the invention, it is also an object of the present invention the cosmetic use of the compounds of formula (I) for prevention or treatment of skin aging. In particular, they can be used for preventing or suppressing the formation of wrinkles, in particular wrinkles caused by reduced level of heparanase sulfate in the epidermal basal membrane.

The compounds of the invention are particularly suitable for such cosmetic applications due to their low cytotoxicity.

It is also an object of the invention, a cosmetic composition comprising the compound of formula (I) and cosmetically acceptable excipients and/or vehicles. Such excipients or vehicles can be chosen by the skilled person among the cosmetic ingredients commonly used in the field. In particular, they are components commonly used in external preparations. Such cosmetic composition can be used in the prevention or suppression of wrinkles or in any other anti-aging applications. It is preferably applied and administered topically.

The following examples further illustrate the invention.

### EXAMPLES

Three different synthetic pathways (A, B, C) have been followed by exploiting the reactivity of different functional groups present in the N-Acetyl heparin glycol split molecule as shown in the following scheme 15. Each pathway required different activations of the spacer bearing the biotin moiety that are described in the following examples.

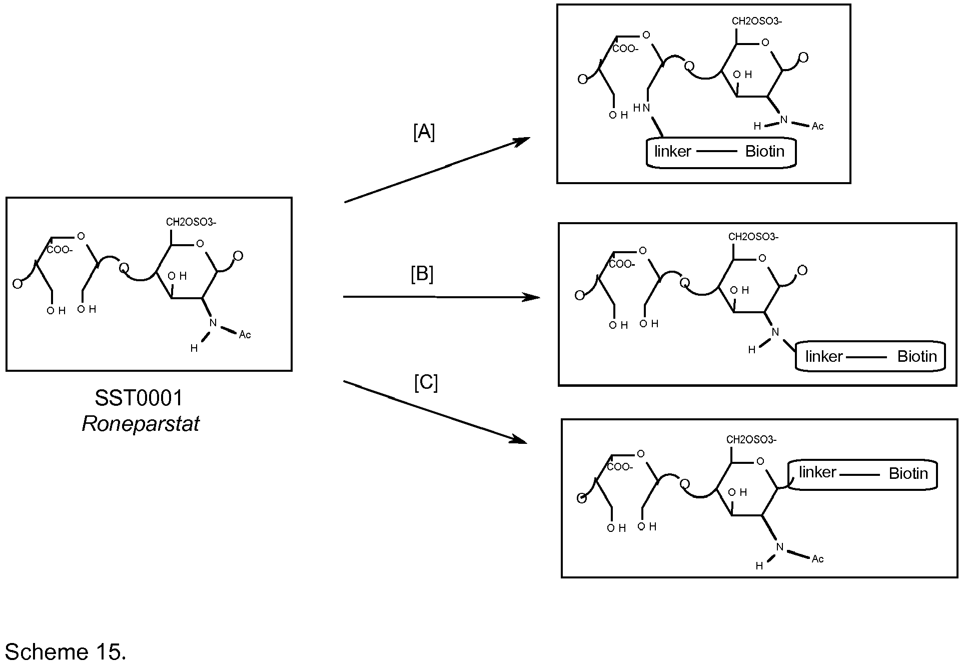

### General procedure of NMR analysis

Spectra were recorded at 25°C on a Bruker Avance 500 equipped with 5-mm-TCI cryoprobe. Integration of peaks area or volumes in the spectra was using standard Bruker TopSpin 2.0 software. The degree of N-desulfation was determined by ¹³C-NMR (Naggi A., et al. 2001, "Generation of anti-factor Xa active, 3-0-sulfated glucosamine-richsequences by controlled desulfation of oversulfated heparins." Carbohydr. Res. 336, 4, ,283-290).

### Example 1

### Synthesis of biotin-amine linkers [A]

### 1.1. Biotin-Peg-NH-Boc S2168 (other batch S2166)

The commercial product, 15-(t-Butyloxycarbonyl) amino-4,7,10,13-teraoxa-pentadecanoic acid (Boc-NHPeg4-OH; 358 mg, 0.98 mmol) was dissolved in 6.5 ml of CH₂Cl₂ and cooled to 0°C in an ice-water bath. N-hydroxy succinimide (NHS; 147 mg, 1.27 mmol) and Dicyclohexylcarbodiimide (DCC; 262 mg, 1.3 mmol) were added to the reaction mixture and stirred at 0°C for 10 min. and then 2 h at room temperature, monitoring the disappearance of the acid by TLC analysis. The reaction mixture was diluted with 10 ml of CH₂Cl₂, then cooled and filtered to remove the DCU and the organic phases were gently concentrated to % of the initial volume (4 ml). Next, to the activated solution, 5-(Biotinamido)pentylamine ,(322 mg, 0.98 mmol) dissolved in 0.5 ml of DMF and 165 µl of TEA (0.98 mmol) was added and the reaction mixture was stirred at room temperature overnight. The organic solvent was evaporated under high vacuum and the obtained crude product was dissolved in with CH₂Cl₂ (40 ml). It, after workup by washing with 0.1N HCl, water, NaHCO₃ 5%, (7 ml) and with brine (7 ml), was dried over anhydrous Na₂SO₄, filtered and concentrated. Finally it was purified by silica gel chromatography (CH₂Cl₂/MeOH 9:1) to give a white solid (428mg, yield 65 %). The structure was confirmed by ¹H-NMR spectroscopy analysis (500 MHz): δ [ppm, D₂O (d₂)], 4.61 (dd, 1 H biot.); 4.42 (dd, 1 H, biot.); 3.32 (1 H, m, -OCH₂CH₂CO-); 3.68 (12H, s, - CH₂CH₂O-) 3.58 (2H, m, -CH₂NHCO-); 3.28 (t, 2H 2.97 (dd, biot.); 2.75 (1H, dd, biot.)2.15 (2H, t, -CH₂CO₂NH-); 1.76-1.3 (22H, m, -CH₂-, biot + -OtBu); and Mass spectroscopy Q-Tof (m/z); [M+Na⁺]= 698.36; [M+K⁺] =714.32 and was used without further purification.

### 1.2. Biotin-Peg-NH₂ x TFA S2293 (other batches S2166, 2295)

Compound **S2168** (Biotin-Peg-NHBoc, 69 mg, 0.10mmol) was dissolved in 0.25 ml of CH₂Cl₂ to the solution was added an equal volume of trifluoroacetic acid (TFA, 0.25 ml), and the mixture kept under magnetic stirring for 45 min. until total conversion. Then solvent was removed. With the purpose to completely remove TFA, co-evaporations first with water and then with methanol (x3) were performed. The obtained white solid, deprotected Biotin-Peg3-NH₂, (74 mg, yield 100 %), was used immediately without further purification in the next step. The structure was confirmed by ¹H-NMR spectroscopy analysis (500 MHz): δ [ppm, D₂O (d₂)], 4.61 (1 H, dd, biot.); 4.42 (1 H, dd, biot.); 3.78 (4H, t, ,-CH₂CH₂O-); 3.68 (12H, t, - CH₂CH₂O-) 3.32 (1 H, m, (dd, biot.); ); 3.21 (t, 2H, -CH₂CH₂NH₂), 3.18 (4H, t, - OCH₂CH₂NHCO-), 2.97 (dd, biot.); 2.78 (1H, dd, biot. )2.50 (2H, t, -CH₂CH₂CO₂NH-); 2.22 (2H, t, -CH₂CO₂NH-); 1.76-1.3 (12H, m, -CH₂-, biot.); and Mass spectroscopy Q-Tof (m/z) [M+H⁺]= 355.16; [M+Na⁺]= 377.16; [M+K⁺] =393.13 and was used without further purification.

### 1.3. NH₂-GGG-(SGGG)₂-Biotin

### FmocNH-GGG-[S(OtBu)GGG]₂-OH, peptide S2588 (other batch S2450)

The oligopeptide Fmoc-NH-GGG-(GGGS)₂-OH, were produced by chemical synthesis using Fmoc-chemistry on solid phase. The general principle of this method is based on repeated coupling and deprotection cycles using the 9H-fluoren-9-ylmethoxycarbonyl, as a protective group of the N-terminal end of growing peptides. The coupling reaction of this preparation was conducted under the same condition. H-Gly-Trt (2-Cl) resin (1.78 g, substitution: 0.56 mmol/g) was swollen in DMF (10 ml) for over 30 min. before use. Each Fmoc-Amino acid (2 mmol, 2equiv) was coupled by coupling reagent HOBt and TBTU (2 mmol, 2 equiv) and DIEA (4 mmol, 4 equiv) in 17 ml of DMF with Microwave-heating assisted, irradiated at 30 W for 5 min. The Fmoc- group was removed using 20% piperidine in DMF (15 ml) under MW irradiation (25 W for 2 min.). Each coupling and deprotection was conducted only one except second Fmoc-Ser(OtBu)-OH and was monitored by Kaiser test. Following completion of the sequence, the linear peptide (eleven amino acid) was cleaved from resin by treatment with 1% TFA in CH₂Cl₂ (2 min. repeated for 10 times), and concentrated at reduced pressure. With the purpose to completely remove all TFA, co-evaporations first with water and then with methanol (x3) were performed to afford the crude white solid product of protected linear precursor S2588 Fmoc-NH-GGG[S(OtBu)GGG]₂-OH (240 mg, yield 24%). It was used immediately without further purification in the next step. The structure was confirmed by ¹H-NMR (500 MHz): δ [ppm, DMSO (d₆)], 8.21-7.85 (10H, m, -NHCO- biot.); 7.90 (2H, d, Fmoc-), 7.71 (2H,d, Fmoc-); 7.51 (1H, m, Fmoc-), 7.41 (2H, t, Fmoc-), 7.33 (2H, t, Fmoc-); 4.34 (2H, dd, Cα Ser), 4.29 (2H, d, Fmoc-), 4.22 (1H, t, Fmoc-), 3.81-3.72 (18H, m, Gly), 3.51 (4H -CH₂OH, Ser), 1.11 (18H, s, -OtBu); and Mass Maldi-Tof (m/z), [M+Na⁺]= 1062.16;[M+K⁺] =1078.14

### 1.4. FmocNH-GGG-[(S(OtBu)GGG)]₂-Biotin S2600 (other batch S2599)

The crude compound **S2588,** FmocNH-GGG-(S(OtBu)GGG)₂-OH ( 233 mg, 0.22mmol) was dissolved in 6 ml of DMSO. The carboxylic acid of substrate was activated by HCTU (97 mg, 0.23 mmol) and DIEA (83µl 0.47 mmol) for 10 min. at room temperature. The activated solution was then added into commercial compound, 5-(Biotinamido)-pentylamine (77 mg, 0.23 mmol) was dissolved in 0.5 ml of DMSO and the resulting solution was stirred at room temperature overnight, following disappearance of the acid on TLC (CH₂Cl₂/MeOH 7:3) analysis. Next, the solvent was evaporated under high vacuum and with the purpose to completely remove DMSO, co-evaporations with water (x3) were performed to obtain a white solid residue. To remove excess reagents, it was washed several times with CH₂Cl₂ and finally with ether. Then, the crude compound was dried under high vacuum and used immediately without further purification in the next step. The structure was confirmed by Maldi-Tof analysis (m/z) [M+Na⁺] = 1372.56; [M+K⁺] =1388.54

### 1.5. NH₂-GGG-[S(OtBu)GGG]₂-Biotin S2608 (other batches S2601)

The crude compound **S2600,** FmocNH-GGG-[(S(OtBu)GGG)]₂-Biotin, (220 mg, 0.21mmol) was dissolved in 10 ml of DMSO, Diethylamine (Et₂NH, 654µl, 6.3 mmol) was added and the reaction mixture stirred at room temperature overnight until total removal of Fmoc- group. Then, the solvent was evaporated under high vacuum and, with the purpose to completely remove DMSO, co-evaporations with water (x4) were performed to obtain a white solid residue. To remove excess reagents and dibenzofulvene adduct, it was washed several times with CH₂Cl₂ and finally with ether. The crude compound was dried under high vacuum and used without further purification in the next step. The structure was confirmed by Maldi-Tof analysis (m/z) [M+Na⁺] = 1150.64; [M+K⁺] =1166.61.

### 1.6. NH₂-GGG-(SGGG)₂-Biotin S2611 (other batches S2450)

The crude compound **S2601** NH₂-GGG-[(S(OtBu)GGG)]₂ , 218 mg, 0.21mmol) was dissolved in 10 ml of TFA / DCM (8/2) mixture and it was stirred for 2 h until total conversion. After that, solvent was removed. With the purpose to completely remove all TFA, co-evaporations with toluene (x3) were performed and obtain white solid Biotin-AA- NH₂ deprotected (185 mg, yield 91 %),. The crude compound was recovered, lyophilized, and purified by size exclusion chromatography on G-10 Sephadex using water/ethanol (9:1) as an eluent. The final product was confirmed by Maldi-Tof (m/z) [M+Na⁺] = 1038.33; [M+K⁺]=1054.30 and ¹H-NMR analysis (500 MHz): δ [ppm, D₂O (d₂)], 4.61 (dd, 1 H biot.); 4.52 (2H, q, Cα Ser), 4.42 (1 H,dd, biot.); 4.08 (18H, m, Gly), 3.91 (4H -CH₂OH Ser), 3.33 (1H, m, biot.); 3.25-3.14 (4H, m,-CH₂NHCO-); 3.01 (1 H, dd, biot.), 2.78 (1 H, d, biot.), 2.25 (2H, t, -CH₂CO₂NH-biot); 1.76-1.3 (12H, m, -CH₂-, biot.).

### Example 2

### Synthesis of carboxyl-activated linkers [B]

### 2.1 Biotin-OSu-activated: S2204 (other batches S2223)

The commercial product 6-Biotinylamino-hexanoic acid (Biotin-Ahx-OH, 101 mg, 0.28 mmol) was dissolved in 4 ml of DMF (light suspension). N-hydroxy succinimide (NHS, 36 mg, 0.31 mmol) and N-(3-Dimethylaminopropyl)-N' ethyl-carbodiimide (EDC x HCl, 59 mg, 0.31 mmol.) were added to reaction mixture and stirred at room temperature for 2 days following disappearance of the acid on TLC analysis. Then, the organic solvent was evaporated under high vacuum and the obtained residue was dissolved in CH₂Cl₂ (20 ml) and rapidly washed with 0.1 N HCl solution to remove excess reagents. The combined organic phase were washed with brine (20 ml), dried over anhydrous Na₂SO₄, filtered and concentrated to give a white solid Biotin-OSu activated (91 mg, yield 71 %), used immediately without further purification.

### 2.2 Biotin-Peg₃-OtBu-acid: S2221 (other batches S2205, S2166)

The commercial product 6-Biotinylamino-hexanoic Acid (Biotin-Ahx-OH, 247 mg, 0.69 mmol) was dissolved in DMF (10 ml, light suspension). The carboxylic acid of substrate was activated by HCTU (347 mg, 0.84 mmol) and DIEA (298µl 1.7 mmol) for 10 min. at room temperature. The activated solution was then added to commercial compound tBuO-Peg3-NH₂ (98.6 mg, 0.76 mmol) solution (1ml dry DMF). Then, others DIEA (92 µl, 0.5 mmol) was added and reaction mixture was stirred at room temperature for 2h. The organic solvent was evaporated under high vacuum and the crude product obtained was dissolved in with CH₂Cl₂ (20 ml), washed with NaHCO₃ 5%, (7 ml) and with brine (7 ml), dried over anhydrous Na₂SO₄, filtered, concentrated and purified by silica gel chromatograph (CH₂Cl₂/MeOH 9:1) to give a white semi solid compound (401 mg, yield 87 %) and was used without further purification. The structure was confirmed by ¹H-NMR spectroscopy analysis (500 MHz): δ [ppm, D₂O (d₂)], 4.61 (dd, 1H biot.); 4.42 (dd, 1H, biot.); 3.78 (4H, t, -OCH₂CH₂CO-); 3.69 (12H, s, - CH₂CH₂O-) 3.33 (1H, Biot.), 3.21 (t, 2H, -OCH₂CH₂NH₂x TFA); 3.18 (t, 4H ,-CH₂CH₂NHCO-); 3.01 (1 H, dd, biot.); 2.759 (1 H, d, biot.) 2.5 (2H, t, -CH₂CO₂NH-); 2.25 (2H, t, -CH₂CO₂NH-); 1.79-1.31 (12H, m, -CH₂-, biot .); and Mass spectroscopy Q-Tof (m/z) [M+H⁺]= 355.16; [M+Na⁺]= 377.16; [M+K⁺] =393.13.

### 2.3 Biotin-Peg₃-OH-acid: S2228 (other batches S2217; S2215)

Compound **S2221** (Biotin-Peg3-OtBu, 391 mg, 0.59 mmol) was dissolved in 1.2 ml of CH₂Cl₂ and an equal volume of trifluoroacetic acid (TFA, 1.2 ml) was added. The mixture was kept under magnetic stirring for 1 h until total conversion. Then, solvent was removed. With the purpose to completely remove TFA, co-evaporations with water first and then toluene (x3) were performed and a white solid Biotin-Peg3-deprotected (374 mg, yield 100 %), was obtain. It was used without further purification. The structure was confirmed by ¹H-NMR analysis (500 MHz): δ [ppm, [D₂O (d2)]; 4.6 (dd, 1 H, biot.); 4.39 (dd, 1 H, biot.); 3.81 (t, 2H,-OCH₂CH₂NH, Peg), 3.65 (t, 2H, -OCH₂CH₂CO-), 3.67 (12H, s, - CH₂CH₂O-),3.40 (2H, t,-OCH₂CH₂NHCO-); 3.33 (m, 1H, biot.); 3.17 (2H, m, -OCH₂CH₂NHCO-) 3.0 (dd, 1H, biot.) 2.78 (d, 1H,-CH₂CO₂H-); 2.25 (t, 4H, -CH₂CONH); 1.78-1.34 (12H, m, -CH₂-, biot).

### 2.4 Biotin-Peg₃-OSu-activated: S2230 (other batches S2218; S2216)

Compound **S2228** (Biotin-Peg3-OH, 364 mg, 0.60 mmol) was dissolved in 10 ml of DMF (light suspension). N-hydroxy succinimide (NHS, 70 mg, 0.6 mmol) and N-(3-Dimethylaminopropyl)-N' ethylcarbodiimide (EDC x HCl, 115 mg, 0.6 mmol.) were added to reaction mixture and stirred at room temperature for 2 days monitoring the disappearance of the acid by TLC analysis. Then, the organic solvent was evaporated under high vacuum and the residue obtained was dissolved in CH₂Cl₂ (35 ml) and rapidly washed with 0.1 N HCl solution. The combined organic phase were washed with water (20 ml), dried over anhydrous Na₂SO₄, filtered and concentrated to give a white solid, Biotin-Peg3-OSu activated (401 mg, yield 95 %), used immediately without further purification.

### Example 3

### Synthesis of Biotin-azide Linkers [C]

### (Preparation of triazole terminated biotin linker)

To introduce primary amino groups at heparin reducing ends, samples conjugate with alkyne (G-alkyne) were synthesized with different methods. This step introduces a primary amino group on the reducing end of heparin chain, that is further modified with amino reactive linkers or biotinylated heparins. In solution, the reducing end residues of heparin chains are in an equilibrium state between the cyclic and the acyclic form. In the acyclic form the aldehyde can form an imine with one primary amino group of 2 amino propargyl. Because this imine is not stable enough, it is best stabilized by its reduction to a secondary amine using sodium cyanoborohydride.

### 3.1 Biotine-cadaverine azide S2493 [Biotin-N₃]

To prepare TfN₃, NaN₃ (394 mg, 6.05 mmol) was dissolved in water (1ml) and added to 1.7 ml of CH₂Cl₂. Tf₂O (175 µl, 1.2 mmol) was added dropwise to the mixture, cooled at 0°C and vigorously stirred. Then the mixture was stirred at 0°C for additional 2h. The organic phase was separated and the aqueous phase was extracted with CH₂Cl₂ (3 ml). The combined organic phases were washed with saturated NaHCO₃ (2 ml) and use immediately. Commercial Biotine cadaverine (5-Biotinamido-pentyamine), (200 mg, 0.61 mmol) was dissolved in 2 ml of water, potassium carbonate (K₂CO₃, 98 mg, 0.71 mmol) and CuSO₄ hydrate (1.4 mg 5.6 µmol in 0.5 ml water) were added to the solution consecutively. Then freshly prepared dichloromethane solution of TfN₃ was added, the solution was brought to homogeneity by adding MeOH (ca.1.5 ml) and was stirred at room temperature for overnight. Organic solvent (MeOH/CH₂Cl₂) was evaporated under vacuum and the remaining aqueous phase washed with CH₂Cl₂ (3 x 3 ml) to remove excess of reagents. Then, the aqueous phase was neutralized with NaHCO₃ 5%, concentrated and purified by silica gel chromatography (CH₂Cl₂/MeOH 96:4) to give a white solid (162 mg, yield 75 %). The crude compound Biotin-N₃ was characterized by ¹H-NMR spectroscopy analysis (500 MHz): δ [ppm, D₂O (d₂)], 4.57 (t, 1H biot.); 4.37 (dd, 1H, biot.); 3.31 (3H, m, biot + -CH₂N₃); 3.17 (2H, m, 2H, -CH₂NHCO-); 2.97 (dd, biot.); 2.75 (1H, dd, biot.)2.15 (2H, t, -CH₂CO₂NH-); 1.76-1.34 (12H, m, -CH₂-, biot); and Mass spectroscopy ESI-Tof (m/z) [M+H⁺]= 355.16; [M+Na⁺]= 377016; [M+K⁺] =393.13 and was used without further purification.

### 4.3 Biotin cadaverine glycine azide S2490 [Biotin-Gly-N₃] (similar preparation S2670)

Compound **Ni1594** (N₃-Gly-OH, 43 mg, 0.45 mmol) was dissolved in DMF (1.5 ml). HCTU (207 mg, 0.5 mmol), DIEA (194µl 1 mmol) were added and the solution stirred for 10 min. at room temperature. The solution of Gly-Azide was added to a solution of commercial compound Biotine-cadaverine (5-Biotinamido)-pentyamine, (200 mg, 0.6 mmol) in dry DMF (1ml). Others DIEA (92 µl, 0.5 mmol) was added to the mixture that was stirred at room temperature overnight. The organic solvent was evaporated under high vacuum and the obtained crude product was dissolved in water (3 ml). The solution was acidified with 0.1 N of HCl. The aqueous phase was washed with CH₂Cl₂ (3 x 10 ml) to remove excess reagents. Finally, the aqueous phase was neutralized with NaHCO₃ 5%, concentrated and purified by silica gel chromatograph (CH₂Cl₂/MeOH 95:5) to give white solid (191 mg, yield 84 %). The Biotin-Gly-N3 was characterized by ¹H-NMR analysis (500 MHz): δ [ppm, [CD₃DO (d4) ]; 4.54 (dd, 1 H, biot.); 4.32 (dd, 1 H, biot.); 3.75 (m, 2H, -CH₂N₃), 3.25-3.19 (6H, m, -CH₂NHCO- , -CH₂N₃); 2.97 (dd, biot.); 2.75 (1 H, dd, biot.) 2.15 (2H, t, -CH₂CO₂NH-); 1.76-1.34 (12H, m,-CH₂-, biot).

### 3.2 Gly-azide NI1594 [N₃-Gly-OH]

A solution of NaN₃ (455 mg, 7 mmol) in 1.2 ml water was added in ice bath and treated with 2 ml of CH₂Cl₂. The resulting biphasic mixture was stirred vigorously and treated with Tf₂O (192 µl, 1.4 mmol) over period of 5 min. The reaction mixture was stirred in a ice bath for 2h, then the organic phase was separated and the aqueous phase was extracted with CH₂Cl₂ (2 x 2 ml). The combined organic phase were washed with saturated NaHCO₃ (3 ml) and use immediately without further purification. Commercial compound Glycine, (54 mg, 0.7 mmol) was dissolved in 2.5 ml of water and treated with potassium carbonate (K₂CO₃, 145 mg, 1.01 mmol) and CuSO₄ hydrate (1.7 mg 7 µmol in 0.4 ml water) were added to the flask consecutively. Freshly prepared dichloromethane solution of TfN₃ was then added, and the solution was brought to homogeneity by adding MeOH (ca.1.3 ml). The resulting solution was stirred at room temperature overnight. The only organic phase (MeOH/ CH₂Cl₂) was evaporated under vacuum and the remaining aqueous solution was poured into 6 ml of water and the pH value was adjusted to 6 with 0.1 N HCl solution and added to 8 ml of phosphate buffer (0.02M pH=6.2). The acid aqueous phase was washed with AcOEt (4 x 5 ml) and then concentrated to 1/2 of initial volume, acidified around pH ≈2 with 0.1N HCl solution and then extracted with AcOEt (5 x 10 ml). The combined organic phase were washed with brine (20 ml), dried over anhydrous Na₂SO₄, filtered and concentrated to give white liquid (64 mg, yield 90 %). The final product was used without further purification and it structure confirmed by ¹H-NMR analysis (300 MHz): δ (ppm, CDCl₃), 3.97(2H, s, *-CH₂*-) and Mass spectroscopy ESI-Tof(m/z) [M+H⁺]= 102.12

### Example 4

### Synthesis of intermediate heparin

### 4.1 Preparation of N-desulphated heparin: G11976 and G11911

A sample of heparin G3378 (8.01g) dissolved in 60 ml of water and converted in the acidic form with Amberlite IR 120 (H⁺form). An excess of pyridine was added to aqueous solution and the mixture obtained evaporated under reduced pressure; the resulting pyridine salt of the heparin was dissolved in 80ml of a mixture of DMSO /H₂0 (95:5) and stirred at 25°C for overnight, in order to obtain a desulphation degree of about 100%. Then, the solution was diluted with an equal volume of water and it was dialyzed against distilled water in membranes (cut-off 3500Da) for 72 h. Then, product was isolated by evaporation under reduced pressure and lyophilized. The structure of the product, used as such in the next step, was confirmed by ¹³C-NMR and SEC-TDA analysis.

### 4.2 Preparation of N-acetylated heparin: G11983 and G11949

A sample of N-desulphated heparin G11976 (2.5 g) dissolved in 57 ml of NaHCO₃ saturated solution was cooled to 4°C. in a ice-water bath. To the vigorously stirred mixture acetic anhydride (Ac₂O, 2.1ml) was added and the mixture was stirred at 4°C for 30min. During the reaction, pH was controlled and maintained at about 8 by adding sodium hydrogen carbonate. Finally, other 2.1 ml of acetic anhydride was added and the mixture was maintained at 4°C for another 1.5 h. Then, the solution was diluted with an equal volume of water and it was dialyzed against deionised water in membranes (cut-off 3500Da) for 72 h. Then, product was isolated by evaporation under reduced pressure and lyophilized. The structure of final product, used as such in the next step, was confirmed by ¹³C-NMR and SEC-TDA analysis.

### 4.4 Preparation of N-acetylated-gs-heparin-Aldehyde: G11954 (similar preparation G11026)

A sample of N-acetylated heparin G11949 (1.15 g) dissolved in 33.5 ml of water and cooled to 4°C, was added to an equal volume of a 0.2 M NalO₄ (33.5 ml) solution and kept under magnetic stirring in the dark for 20 h. The excess of periodate was quenched by adding ethylene glycol (3.01 ml) and after 1 hr the reaction mixture was desalted by dialysis against distilled water in membrane cut-off 3500 Da at 4°C in the dark for 16 hrs. The retentate was recovered, gently concentrated to 1/2 initial volume and lyophilized. The final product (1.04 g, yield=90%) was used as such in the next step.

### 4.5 Preparation of N-desulphated-gs heparin: G12065

A sample of N-desulphated heparin G11976 (1.2 g) dissolved in 35 ml of water and cooled to 4°C, was added to an equal volume of a solution 0.4 M NalO₄ (33.5 ml). The pH value was adjusted to 3.5 with pyridine (10% v/v, about 0.5 ml) and kept under magnetic stirring in the dark for 6 h. Then, the oxidized heparin was precipitated by the addition of 160 ml of ethanol (96% v/v) containing 3% (w/v) of sodium acetate. The precipitate was collected by centrifugation (12.000 rpm) and the precipitated was washed several times with ethanol to remove excess of reagents and sodium acetate. To crude residue dissolved in 33 ml of water was added NaBH₄ (731 mg) in several portions, and the mixture was stirred for 3 hrs at 25°C. Next, under stirring at 4 °C the pH value was adjusted brought to 4 with 5% (v/v) acetic acid solution to quench the NaBH₄ excess. After ten minutes stirring, the mixture was neutralized with 0.1 N NaOH. After dialysis against deionised water in membrane cut-off 3500 Da at room temperature, concentration under reduced pressure and freeze drying the final compound was obtained (1.05g yield=87%). Content of gs-residues was confirmed by ¹H-HSQC and molecular weight evaluated by SEC-TDA analysis.

### 4.6 N-acetylated heparin-alkyne Reducingend-Functionalized : G11993 and G11949

A sample of N acetylated Heparin G11983 (1.21 g, 80µmol) was solubilized in 15 ml of phosphate buffer 15 mM pH=5.5 in one neck flask under magnetic stirring at room temperature. Then, 2 amino propargyl (266 mg, 2.9 mmol) was added to the reaction mixture and the solution was heated up to 50°C and kept under magnetic stirring for 3 h. Then sodium cyanoborohydride (130 mg, 2.09 mmol) was added and the mixture was incubated at 50°C for additional 24 h. A further 20eq. 130mg NaCNBH₃ was added and the mixture heated for another 1 day. The reaction mixture was diluted with water and dialyzed against distilled water at room temperature for 2 days using a 3500 molecular weight cut-off (MWCO) dialysis membrane. The retentate was recovered, lyophilized, and purified by alcoholic precipitation followed by size exclusion chromatography on G-10 Sephadex using water/ethanol (9:1) as an eluent. The efficiency of the reductive amination was evaluated measuring the rate of amino group attached on the polysaccharide and anomeric signals by ¹H-NMR.

### Example 5

### N-acetylated-gs-heparin-alkyne Reducingend-Functionalized : G11997

A sample of N acetylated Heparin-alkyne G1193 (1.21 g), dissolved in 32 ml of water and cooled to 4°C, was added to an equal volume of a solution 0.2 M NalO₄, and kept under magnetic stirring in the dark for 20 h. The excess of periodate was quenched by adding ethylene glycol (3.4 ml) and after 1 hr the reaction mixture was desalted by dialysis against distilled water in membrane cut-off 3500 Da at 4°C for 16 hrs. The retentate was treated with NaBH₄ (740 mg), stirred for 3hrs at 25°C then, under stirring at 4 °C the pH value was adjusted brought to 4 with 5% (v/v) acetic acid for quenching the NaBH₄ excess, and after 10 minutes stirring, neutralized with 0.1 N NaOH. Then the solution was dialysed against distilled water in membrane cut off 3500 Da at room temperature, concentration under reduced pressure and freeze dried. The crude compound containing the gs-derivatives were desalted on a Sephadex G-10 column (900 x 25mm) using water/ethanol 9:1 (v/v) as a mobile phase at 2.1 ml/min. and monitoring the absorbance at 210 and 190 nm. After freeze-drying, 1.01g of RO-alkyne was obtained with 85% (w/w) yields. The efficiency of the acetylation reaction was evaluated measuring the acetyl group by ¹H-NMR value.

### Example 6

### Biotinated-Heparin conjugated on Glucosamine unit

### 6.1 N-desulphated-gs-Biotinated-heparin: G12068

To a sample of N-desulphated-gs-heparin G12065 (1.1 g) dissolved in 20 ml of 0.1N NaHCO₃ and cooled to 4°C, Biotin-Peg3-OSu S2230 was added, 320 mg (about 0.25 equiv.) dissolved in 1 ml of DMF and the mixture was stirred at room temperature for 2h. Then a further 128 mg (about 0.1 equiv.) of biotin activated S2230 was added and the mixture stirred for additional 12h. The reaction medium obtained was diluted with 10 ml of NaCl 2M and dialyzed against distilled water at room temperature for 1 day using a 3500 molecular weight cut-off (MWCO) dialysis membrane. The retentate was concentrated up to about 15 ml and purified by alcoholic precipitation with addition of 30 ml of ethanol (96% v/v). The precipitate was collected by centrifugation (12.000 rpm) and washed several times with ethanol. The supernatant was discarded, the precipitate was re-dissolved in 15 ml of water, and the process was repeated twice. The final compound containing the biotin-gs-conjugated was caracterized by ¹H-HSQC and SEC-TDA analysis and used as such in the next step.

### 6.2 N-Acetylated-gs-Biotinated- heparin: G12072 (similar preparation G11487F with DS=6.4%)

A sample of N- desulphated-gs-heparin G12068 (0.85g) dissolved in 23 ml of saturated solution NaHCO₃ was cooled to 4°C. in a ice-water bath. To the vigorously stirred mixture was added acetic anhydride (Ac₂O, 1.1ml) and the mixture was stirred at 4°C for 30 min. During the reaction, pH was controlled and maintained at about 8 by adding sodium hydrogen carbonate. Finally, other 1.1 ml of acetic anhydride was added and the mixture was maintained at 4°C for another 1.5 h. Then, the solution was diluted with an equal volume of water and it was dialyzed against distilled water in membranes (cut-off 3500Da) for 2 days. Then, product was isolated by evaporation under reduced pressure and lyophilized to obtain the desired biotinylated-gs-heparin G12072, which was purified by gel permeation chromatography (shephadex G-10) using water/ethanol (9:1) as an eluent. The final product (720 mg, yield=84%) was caracterized by ¹H-HSQC and SEC-TDA (MW=14100) analysis. The amount of conjugation of linker biotinated was evaluated by ¹H-NMR (DS= 6.7 for disaccharide)

### Example 7

### Biotinated-Heparin conjugated on glycol split unit

### 7.1 N-Acetylated-gs-Biotinated- heparin: G11980 (other batch G11493F with DS 6.6 % (SST0761NA1)

A sample of N-acetylated-gs-heparin-Aldehyde G11954 (0.95g) was solubilized in 15 ml of phosphate buffer 15 mM pH=5.5 in one neck flask under magnetic stirring at room temperature. Then, **NH₂-GGG-(SGGG)₂-Biotin** S2611, 210 mg (about 0.25 equiv) was added in 1 ml of phosphate buffer and the reaction mixture was heated up to 40°C and kept under magnetic stirring for 1 h. Then sodium cyanoborohydride (NaCNBH₃, 260 mg, 20 equiv.) was added and the mixture was maintained at 40°C for an additional 24 h. After cooling to 25°C, further 157 mg of NaBH₄ (20 equiv) was added and the reaction mixture was maintained at room temperature for an additional 3 h. Then, under stirring at 4°C the pH value was adjusted to 4 with 5% (v/v) acetic acid for quenching the NaBH₄ excess, and after 10 minutes stirring, neutralized with 0.1 N NaOH. The obtained reaction medium was diluted with 10 ml of NaCl 2M and dialyzed against distilled water at room temperature for 1 day using a 3500 molecule weight cut-off (MWCO) dialysis membrane. The product was isolated by evaporation under reduced pressure and lyophilized to obtain the desired biotinylated-gs-heparin G12072, which was purified by gel permeation chromatography (shephadex G-10) using water/ethanol (9:1) as an eluent. The final product (about 810 mg, yield=84%) was caracterized by ¹H-HSQC and SEC-TDA analysis. The amount of conjugation of linker biotinated was evaluated by ¹H-NMR (DS=3.01 % for disaccharide)

### Biotinated-Heparin conjugated on glycol split unit with different degree of substitution:

### 7.2 N-Acetylated-gs-Biotinated- heparin: G11046 (other batches G11034; G11018A)

A sample of N-acetylated-gs-heparin-Aldehyde G11026 (60mg) was dissolved into 1.5 ml of phosphate buffer 15mM pH=5.5 in one neck flask under magnetic stirring at room temperature. Next, commercial product **Biotin-NH₂** 8.2mg (about 0.5 equiv) was added in 100 µl of phosphate buffer and the reaction mixture was heated up to 40°C and kept under magnetic stirring for 1 h. Then sodium cyanoborohydride (NaCNBH₃, 15.6 mg, 20 equiv.) was added and the mixture was maintained at 40°C for an additional 24 h. After cooling to 25°C, further 10.5mg of NaBH₄ (20 equiv) was added and the reaction mixture was maintained at room temperature for an additional 3 h. Then, under stirring at 4°C the pH value was adjusted to 4 with 5% (v/v) acetic acid for quenching the NaBH₄ excess, and after 10 minutes stirring, neutralized with 0.1 N NaOH. The reaction medium obtained was diluted with 3 ml of NaCl 2M and dialyzed against distilled water at room temperature for 1 day using a 3500 molecule weight cut-off (MWCO) dialysis membrane. The product was isolated by evaporation under reduced pressure and lyophilized to obtain the desired biotinylated-gs-heparin G11046, which was purified by gel permeation chromatography (shephadex G-10) using water/ethanol (9:1) as an eluent. The final product (about 54 mg, yield=90%) was caracterized by ¹H-HSQC and SEC-TDA analysis (MW= 16000). The amount of conjugation of linker biotinated was evaluated by ¹H-NMR (DS=12% for disaccharidic units)

### 7.3 N-Acetylated-gs-Biotinated- heparin: G11034 (other batches G11046; G11018A)

A sample of N-acetylated-gs-heparin-Aldehyde G11026 (110mg) was dissolved into 2.3 ml of phosphate buffer 15mM pH=5.5 in one neck flask under magnetic stirring at room temperature. Then, commercial product **Biotin-NH₂** 30 mg (about 1 equiv) was added in 100 µl of phosphate buffer and the reaction mixture was heated up to 40°C and kept under magnetic stirring for 1 h. Then sodium cyanoborohydride (NaCNBH₃, 28.5 mg, 20 equiv.) was added and the mixture was maintained at 40°C for an additional 24 h. After cooling to 25°C, further 12 mg of NaBH₄ (20 equiv) were added and the reaction mixture was maintained at room temperature for additional 3 h. Then, under stirring at 4°C the pH value was adjusted to 4 with 5% (v/v) acetic acid for quenching the NaBH₄ excess, and after 10 minutes stirring, neutralized with 0.1 N NaOH. The obtained reaction medium was diluted with 3.5 ml of NaCl 2M and dialyzed against distilled water at room temperature for 1 day using a 3500 molecule weight cut-off (MWCO) dialysis membrane. The product was isolated by evaporation under reduced pressure and lyophilized to obtain the desired biotinylated-gs-heparin G11034, which was purified by gel permeation chromatography (shephadex G-10) using water/ethanol (9:1) as an eluent. The final product (about 94 mg, yield=85%) was characterized by ¹H-HSQC and SEC-TDA analysis (MW= 17000). The amount of conjugation of linker biotinated was evaluated by ¹H-NMR value (DS = 41% for disaccharidic units).

### 7.4 N-Acetylated-gs-Biotinated- heparin: G11047 (other batch G11037)

A sample of N-acetylated-gs-heparin-Aldehyde G11026 (51 mg) was dissolved into 1.1 ml of phosphate buffer 15mM pH=5.5 in one neck flask under magnetic stirring at room temperature. Then, **Biotin-Peg-NH₂ S2293** 8.2 mg (about 0.5 equiv), in 100 µl of phosphate buffer, was added and the reaction mixture was heated up to 40°C and kept under magnetic stirring for 1 h. Then sodium cyanoborohydride (NaCNBH₃, 14.6mg, 20 equiv.) was added and the mixture was maintained at 40°C for an additional 24 h. After cooling to 25°C, further 8.5mg of NaBH₄ (20 equiv) was added and the reaction mixture was maintained at room temperature for additional 3 h. Then, under stirring at 4°C the pH value was adjusted to 4 with 5% (v/v) acetic acid for quenching the NaBH₄ excess, and after 10 minutes stirring, neutralized with 0.1 N NaOH. The obtained reaction medium was diluted with 3 ml of NaCl 2M and dialyzed against distilled water at room temperature for 1 day using a 3500 molecule weight cut-off (MWCO) dialysis membrane. The product was isolated by evaporation under reduced pressure and lyophilized to obtain the desired biotinylated-gs-heparin G11047, which was purified by gel permeation chromatography (shephadex G-10) using water/ethanol (9:1) as an eluent. The final product (about 41 mg, yield=80%) was confirmed by ¹H-HSQC and SEC-TDA analysis (MW= 18700). The amount of conjugation of linker biotinated was evaluated by ¹H-NMR (DS = 21% for disaccharidic units)

### 7.5 N-Acetylated-as-Biotinated- heparin: G11037 (other batch G11047)

A sample of N-acetylated-gs-heparin-Aldehyde G11026 (120mg) was dissolved into 2.5 ml of phosphate buffer 15mM pH=5.5 in one neck flask under magnetic stirring at room temperature. Then, **Biotin-Peg-NH₂ S2293** 69mg (about 1 equiv) , in 200µl of phosphate buffer, was added and the reaction mixture was heated up to 40°C and kept under magnetic stirring for 1 h. Then sodium cyanoborohydride (NaCNBH₃, 31 mg, 20 equiv.) was added and the mixture was maintained at 40°C for an additional 24 h. After cooling to 25°C, further 19 mg of NaBH₄ (20equiv) was added and the reaction mixture was maintained at room temperature for an additional 3 h. Then, under stirring at 4°C the pH value was adjusted to 4 with 5% (v/v) acetic acid for quenching the NaBH₄ excess, and after 10 minutes stirring, neutralized with 0.1 N NaOH. The reaction medium obtained was diluted with 3 ml of NaCl 2M and dialyzed against distilled water at room temperature for 1 days using a 3500 molecule weight cut-off (MWCO) dialysis membrane. The product was isolated by evaporation under reduced pressure and lyophilized to obtain the desired biotinylated-gs-heparin G11037, which was purified by gel permeation chromatography (shephadex G-10) using water/ethanol (9:1) as an eluent. The final product (about 95 mg, yield=80%) was caracterized by ¹H-HSQC and SEC-TDA analysis (MW= 21200). The amount of conjugation of linker biotinated was evaluated by ¹H-NMR (DS = 28% for disaccharidic units).

### Example 8

### Biotinated-Heparin conjugated on reducing terminus unit

Compounds G12005, G11506F and G11510F triazole derivate were synthesized by "click" chemistry approach.

### 8.1 Biotinated-triazole-heparin Reducing end-Functionalized: G12005 (other batch G11506F) (SST0764NA1)

A sample of N-acetylated-gs-heparin-alkyne G11997 (1.01 g, 0.067 mmol) was solubilized in a water/t-BuOH mixture (12 ml 70:30 v/v %) and maintained under magnetic stirring for 10 min. Compound S2670 - the same as S2490 - (54 mg, 2 eqs.) was previously solubilized in a water/t-BuOH mixture (1 ml, 1;1) and then added dropwise to the reaction mixture. Sodium ascorbate (300 µl sol. Aqueous 0.2M, 1 equiv) and CuSO₄ x5H₂O (300 µl sol. Aqueous 0.1 M, 0.5 equiv) were added, and the reaction mixture was stirred at room temperature for 2 h. Finally, other 2 equiv of azide and the respective salts was added and the mixture was maintained at room temperature for another 4 h. The aqueous mixture was dialyzed against distilled water for 48 h using a dialysis membrane with MWCO of 3500 Da. The retentate was recovered and concentrated to 15-20 ml and purified from copper with 3 ml of chelex 100 resin and after 30 minutes neutralized with 0.1 N NaHCO₃. After dialysis against distilled water in membrane cut-off 3500 Da at room temperature, concentration under reduced pressure and freeze drying (about 920 mg, yield=87%). The final compound containing the gs-derivatives was confirmed by ¹H-HSQC and SEC-TDA (MW= 14200) analysis. (DS=1.92 % for disaccharidic units)

### 8.2 Biotinated-triazole-heparin Reducing end-Functionalized: G11510F

A sample of N-acetylated-gs-heparin-alkyne G11997 (32mg, 1.9 µmol) was dissolved in a water/t-BuOH mixture (0.8 ml 70:30 v/v %) and maintained under magnetic stirring for 10 min. Compound S2493 (1.2 mg, 2 eqs.) was previously dissolved in a water/t-BuOH mixture (0.8 ml) and then added drop wise to the reaction mixture. Sodium ascorbate (7 µl sol. Aqueous 0.2M , 1 equiv) and CuSO₄ x5H₂O (7 µl sol. Aqueous 0.1 M, 0.5 equiv) were added, and the reaction mixture was stirred at room temperature for 2 h. Finally, other 2 equiv of azide and the respective salts was added and the mixture was maintained at room temperature for another 4 h. The aqueous mixture was dialyzed against distilled water for 48 h using a dialysis membrane with MWCO of 3500 Da. The retentate was recovered and concentrated to 15-20 ml and purified from copper with 3 ml of chelex 100 resin and after 30 minutes neutralized with 0.1 N NaHCO₃. After dialysis against distilled water in membrane cut-off 3500 Da at room temperature, concentration under reduced pressure and freeze drying (about 28 mg, yield=86%). The final compound containing the gs-derivatives was characterized by ¹H-HSQC and SEC-TDA (MW= 9300) analysis. (DS = 3.4% for disaccharidic units).

### Summary of the synthetized compounds

**Table 1**

| Synthetic pathway | Internal code | Example | DS % for disaccharide units | DS for molecule approximated for a molecular weight of 16000 |
|---|---|---|---|---|
| A | G11980 | 7.1 | 3.0 | 1 |
| A | G11046 | 7.2 | 12.0 | 3 |
| A | G11034 | 7.3 | 41.0 | 13 |
| A | G11047 | 7.4 | 21.0 | 7 |
| A | G11037 | 7.5 | 28.0 | 9 |
| B | G12072 | 6.2 | 6.7 | 2 |
| C | G12005 | 8.1 | 1.9 | 0.6 |
| C | G11510F | 8.2 | 3.4 | 1 |

### Biological assays

### Example 9

### Fondaparinux heparanase assay

In order to determinate their activity as potent heparanase inhibitor, compounds were tested in a simple, accurate and robust microplate assay based on the cleavage of the synthetic heparin fragment, the pentasaccharide Fondaparinux (AGA*IA). This assay was originally developed by Hammond and coworkers [Hammond E, Li CP, Ferro V. Development of a colorimetric assay for heparanase activity suitable for kinetic analysis and inhibitor screening. Anal. Biochem. 2010, 396(1), 112-116], where Fondaparinux corresponds to the methyl glycoside of the antithrombin III (ATIII)-activating pentasaccharide sequence of heparin. Fondaparinux is cleaved by heparanase to generate a reducing disaccharide, which can be quantitatively measured via a colorimetric or a fluorescence assay, the last by the fluorescent sensor polymer-H [Schiemann S, Lühn S, Alban S. Development of both colorimetric and fluorescence heparinase activity assays using fondaparinux as substrate. Anal. Biochem. 2012, 427(1), 82-89].

### Materials and methods

The assay measures the appearance of the disaccharide product of heparanase-catalyzed fondaparinux cleavage colorimetrically using the tetrazolium salt WST-1. Briefly, NUNC ELISA 96-well plates were pre-treated with a solution of 4% BSA in phosphate-buffered saline containing 0.05% Tween 20 (PBST), for 2 h at 37°C, then washed three times with PBST, dried by tapping on paper towel and stored at -20°C. Before use, once again, the plates were washed with PBST.

Assay was carried out with 100 µl/well of assay solution containing 40 mM sodium acetate buffer (pH 5.0), 100 µM fondaparinux, 2.5 nM heparanase and serial dilutions of test compounds (tested in triplicate). Plates were sealed with adhesive tape and incubated, in the dark, for 3 h at 37°C, followed by development with 1.69 mM of the tetrazolium salt WST-1 solution in 0.1 M NaOH, for 1 h at 60°C. Then, the absorbance at 560 nm was measured through a microplate reader (Victor 3, Perkin Elmer).

IC50 value for each compound, versus heparanase, was calculated by GraphPad software and results of selected compounds are summarized in the Table 2 below. Finally, the measurements were corrected by subtracting both the reagent background and the inner absorbance value of test compound. N-desulfated, N-acetylated glycol split (gs) heparin (SST0001, also named as Roneparstat; Leadiant Bioscience S.A.; former Sigma-Tau Research, Switzerland) is taken as reference compound.

### Results

Results are shown in the following Table 2.

**Table 2**

| **Compound** | **Family/ linker** | **MW (kDa)** | Glycol Split (RO; %) | | AGA*IA assay (Fondaparinux) |
|---|---|---|---|---|---|
| | | | Residue (% I) | Residue (% G) | IC₅₀ (nM) |
| SST0001 *(Reference compound)* | | ∼ 15 | 22 | 30 | ∼ 5 |
| SST0635NA1 | B/PEG | ∼ 15 | 15 | 19 | ∼ 5 |
| SST0761NA1 | A/GGGS | ∼ 14 | 16 | 11 | ∼ 3 |
| SST0764NA1 | C/(CH₂)₅ | ∼ 14 | 14 | 17 | ∼ 4 |

| | | | | | |
|---|---|---|---|---|---|
| "Glycol-split (RO; %)" means the percentage of the modified uronic acid residues (RO) with respect to the total of the same present in the molecule. In particular, "(% I)" means the percentage of the iduronic acid residues (oxidated and reduced) with respect to the total of the disaccharides present in the polymer. "(% G)" means the percentage of the modified glucuronic acid residues with respect to the total of the disaccharides present in the polymer. The Table 2 above shows that the biotinylated compounds are endowed with a heparanase inhibitory activity in the same range of the reference compound SST0001. | | | | | |

Upon screening for inhibition of heparanase enzymatic activity, selected active compounds were furtherly characterized in vitro, through a suitable cell proliferation assay (SRB Cell Cytotoxicity Assay) for their putative cytotoxic activity against three tumor cell lines, and through specific cellular assay for their putative anti-metastatic activity. According to these data selected compounds were also tested in real-time quantitative PCR.

### Example 10

### Cytotoxicity assay

SRB Cell Cytotoxicity Assay is based on the quantitative staining of cellular proteins by sulforhodamine B (SRB). This assay can be used for high-throughput drug screening (Vichai & Kirkitara, Nature Protocol. 2006, 1, 112). Sulforhodamine B is an anionic aminoxanthene dye that forms an electrostatic complex with the basic amino acid residues of proteins under moderately acid conditions, which provides a sensitive linear response.

To assess a putative antiproliferative activity of the selected compounds, HT1080 (human fibrosarcoma), U87MG (human glioblastoma astrocytoma) and U2OS (human osteosarcoma) cell lines were treated for 72h, in triplicate, with increasing concentrations of heparanase inhibitors. Inhibition of cell proliferation was then measured by the classical colorimetric SRB Cell Cytotoxicity assay. Absorbance was measured by spectrophotometer at a wavelength of 510 nm and cell proliferation was determined as percent with respect to control cells (cells treated with the vehicle alone). EC₅₀ values were then determined by GraphPad Prism.

Results are summarized in the following Table 3, reporting the ability of anti-heparanase agents (test compounds) to reduce tumor growth (cytotoxic effect) on three tumor cell lines, evaluated at three concentrations. Results were expressed as a percentage of growth versus control.

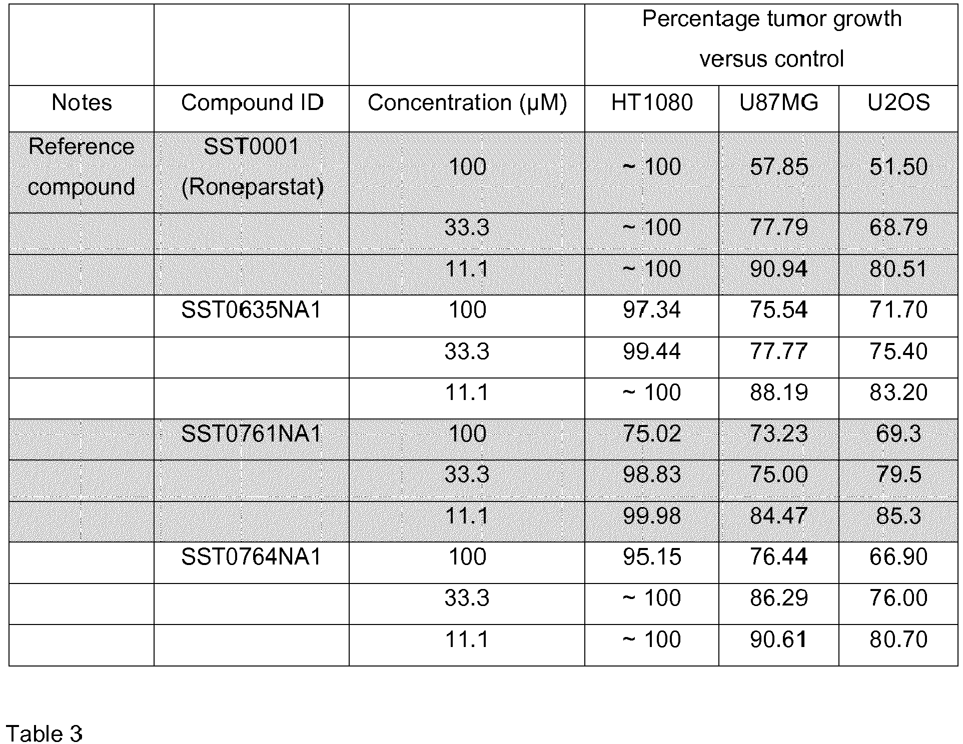

As show in Table 3, none of the selected inhibitors significantly inhibited cell proliferation. In fact, only a limited effect was observed at the highest concentration, with the exception of SST0001 (reference compound) that caused about 50% of cell growth inhibition on two out of three cell lines. These data are very important because they show that compounds of the present invention, at concentrations in which they do not have a relevant interference with cell proliferation, are potent and selective heparanase inhibitors.

### Example 11

### The Invasion Assay

Heparanase inhibitors were then evaluated for their activity on the invasive potential of HT1080 (human fibrosarcoma), U87MG (human glioblastoma astrocytoma) and U2OS (human osteosarcoma) human cell lines, using the Matrigel cell invasion assay which allows to assess the cell invasion ability of malignant and normal cells.

Invasion assay was performed using BioCoat cell culture inserts (BioCoat 8.0µm PET Membrane 24 Well Permeable Supports, Corning). Filters were coated with 100 µl of 300 µg/ml Matrigel dissolved in coating buffer (0.01 M Tris pH 8.0, 0.7% NaCl) (Becton-Dickinson Sciences). DMEM (Dulbecco's Modified Eagle's medium), containing 5% FBS (Fetal Bovine Serum), was added to the lower chambers. Cells were pre-treated with heparanase inhibitors for 24 h, detached and added to the upper chambers in media containing the inhibitors. Cells were allowed to invade for 24 h at 37°C in a CO₂ incubator and then unmigrated cells were removed from the upper chamber with a cotton swab. The invading cells were fixed, stained with 0.1% crystal violet for 10 min at room temperature, photographed and counted under inverted microscope.

Test compounds were analyzed at fixed concentration for several fields, in duplicates. Activity of most interesting compounds was confirmed in a second cell invasion assay. Activity of test compounds was compared to the reference compound. Data were expressed as the percent invasion with respect to cell migration through uncoated insert membrane. SST0635NA1, tested at the fixed concentration (10 µM), was able to significantly block invasion of HT1080 cells (> 50%) and, at lesser extent (< 50%), also of U87MG cells.

### Example 12

### Cell Adhesion Assay

Cell surface, non-integrin molecules such as heparan sulfate proteoglycans (HSPGs) may be involved in the regulation of early adhesive stages of cell-ECM interactions. A cell adhesion assay has been employed to determine if the newly designed heparanase inhibitors were able to interfere with adhesion properties of cancer cells.

For the assay set-up, U87MG (human glioblastoma astrocytoma) cells were treated for 18-20h with test compounds, in complete medium supplemented with 10% FCS. Then, cells were collected, washed in complete medium, and added to matrigel coated wells in triplicate, at previously identified optimal seeding density, and incubated at 37 °C in complete medium for times ranging from 15 minutes to 4h (Goldshmidt et al, FASEB, 2003). After incubation, the wells were washed three times with serum-free medium and the remaining firmly attached cells were stained with crystal violet 0.1 reagent. Optical density was measure at 500 nm by Victor 3 (Perkin-Elmer) plate analyzer. Each experiment was repeated three times.

Results are shown in the following table 4.

**Table 4. Adhesion assay on the selected compounds.**

| Notes | Compound Id | Test compounds adhesion versus basal (100%) | | |
|---|---|---|---|---|
| | | U87MG (human glioblastoma astrocytoma) | | |
| | | 10 µM | 1 µM | 0.1 µM |
| Ref. Cpd | SST0001 | 22.90 | 22.48 | 54.03 |
| | SST0635NA1 | 27.19 | 16.65 | 62.86 |
| | SST0761NA1 | 21.46 | 13.25 | 57.55 |
| | SST0764NA1 | 12.79 | 19.82 | 83.21 |

The three selected compounds inhibited adhesion as well as or greater than the reference compound (SST0001).

Inhibition of adhesion is not directly related to the enzyme inhibition (heparanase activity) so, it should be considered as an adjuvant effect of the tested compounds

### Example 13

### Real-Time Quantitative PCR (qPCR) for measuring mRNA gene-expression of FGF1/2, VEGF, MMP-9, HSPE-1 in genes tumor cell lines

To evaluate the effect of heparanase inhibitors on the tumor transcription of genes encoding for proangiogenic factors, such as FGF1/2, VEGF, MMP-9, HSPE-1, the relative mRNA levels were analyzed by quantitative Real-Time Quantitative PCR. To perform the assay, HT1080 (human fibrosarcoma) cells were cultured in complete medium containing 10%FCS (fetal calf serum). Cells were plated in 24-well plates and treated at fixed concentration (i.e., highest non-toxic concentration) of test compounds. After 24 h, cells were detached, collected and total RNA extracted by RNeasy Mini Kit (QIAGEN). Following reverse transcription step, FGF1/2, VEGF, MMP-9, HSPE-1 mRNA levels, were quantified by qPCR using the advanced Universal SYBR Green Supermix (Bio Rad) and ABI PRISM 7900HT Thermal Cycler System (Perkin Elmer), according to the manufacturer's instructions. Appropriate no-RT and non-template controls were included in each 96-well PCR reaction, and dissociation analysis was performed at the end of each run to confirm the specificity of the reaction. Relative levels of RNA were calculated using the ddCt method. Standard curve and sample data were run in duplicate. In the Table 5 a mean value is reported.

Results are shown in table 5 below.

**Table 5. Real-Time q-PCR analysis of pro-angiogenic genes (10 µM)**

| | Gene Expression (% of control) | | | | |
|---|---|---|---|---|---|
| | FGF-1 | FGF-2 | VEGF | MMP-9 | HPSE-1 |
| | % mRNA | % mRNA | % mRNA | % mRNA | % mRNA |
| Control | ∼ 100 | ∼100 | ∼ 100 | ∼ 100 | ∼ 100 |
| SST0635 | ∼ 78 | ∼74 | ∼ 61 | ∼ 52 | ∼ 67 |
| SST0001 | ∼ 70 | ∼77 | ∼ 43 | ∼ 42 | ∼ 72 |

The biotinylated derivative (SST0635AA1) inhibits gene expression, of various pro-angiogenic factors analysed, to an extent comparable to the parent compound (SST0001).

### Example 14

### Determination of biotin

Biotin activity was determined via the HABA (4'-hydroxyazobenzene-2-carboxylic acid) test (data not shown) while, for a quantitative detection of the biotinylation of G.S. heparin derivatives (biotin/heparin rate), an H-NMR analysis was used.

Results are reported in the following Table 6.

**Table 6**

| SST | Family/ linker | MW (kDa) | Glycol Split (RO; %) | | Biotin/Heparin Rate |
|---|---|---|---|---|---|
| | | | Residue (% I) | Residue (% G) | |
| SST0635NA1 | B/PEG | 9400 | 9.8 | 8.5 | 1.04 |
| SST0761NA1 | A/GGGS | 14100 | 15.8 | 11.1 | 0.71 |
| SST0764NA1 | C/(CH₂)₅ | 14200 | 13.8 | 16.6 | 0.45 |

NMR analysis comfirms the presence of the biotin in the G.S. heparin derivatives and shows that on average each tested derivative is linked to 0.5-1.0 molecule linker-biotin.

### Example 15

### Experimental metastasis. B16-BL6 mouse melanoma cells.

### Materials and methods

Three biotinylated derivatives were studied versus parent compound (SST0001) for their antimetastatic activity. Mice were pre-treated with each compound (50 mg/mouse), administered 1h prior to the vein tail injection of the melanoma cells. The number of melanoma colonies per lung (lung metastases) was determined after 18 days.

### Results

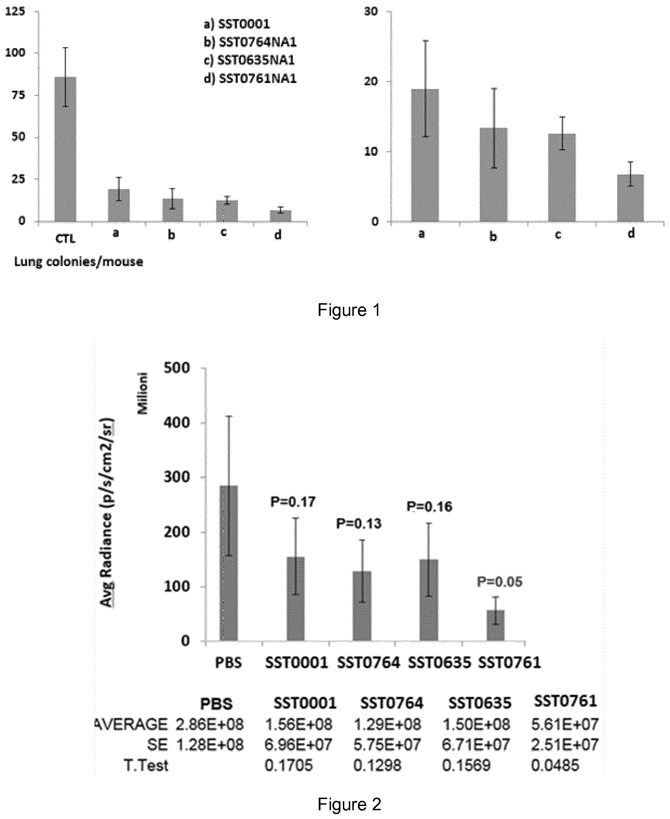

Results are shown in Figure 1.

Three heparin-biotinylated compounds of present invention, in the experimental metastasis model, in mouse (B16-BL6 melanoma), showed an anti-metastatic activity, evaluated as the number of melanoma colonies per lung (lung metastases) after 18 days, in the same range or lesser than the parent compound (SST0001).

### Example 16

### Effect of compounds on CAG myeloma.

The Heparanase Inhibitor SST0001 strongly inhibits the growth of myeloma tumors in vivo (subcutaneous tumors formed by CAG human myeloma cells) by targeting the tumor microenvironment, including a significant inhibition of tumor angiogenesis. [Ritchie JP et al. Clin. Cancer Res. 2011; 17(6), 1382-93]

Here we evaluated the inhibition of CAG myeloma cell growth with three biotinylated compounds (SST0764NA1; SST0635NA1 and SST0761NA1), using SST0001 as control.

### Materials and methods

Briefly, luciferase-labelled CAG myeloma cells (5x106; same cells) were injected subcutaneously into NOD/SCID mice (n=5). Mice were treated with compounds (1000 µg/mouse, injected intraperitoneal twice a day, starting on day 9 of cell inoculation).

Mice were injected with cells on day ONE and the first injection of compounds was given starting on day NINE, twice a day, including weekends. Tumor growth was examined by IVIS imaging prior to the first injection of compounds and once a week thereafter. The experiment was terminated on day TWENTYNINE (Figure 2).

IVIS imaging. Bioluminescent imaging of the luciferase-expressing tumors was performed with a highly sensitive, cooled charge coupled device (CCD) camera mounted in a light-tight specimen box (IVIS; Xenogen Corp., Waltham, MA). Briefly, mice were injected intraperitoneally with D-luciferin substrate at 150 mg/kg, anesthetized and placed onto a stage inside the light-tight camera box, with continuous exposure to isoflurane (EZAnesthesia, Palmer, PA). Light emitted from the bioluminescent cells was detected by the IVIS camera system with images quantified for tumor burden using a log-scale color range set at 5×10⁴ to 1×10⁷ and measurement of total photon counts per second (PPS) using Living Image software (Xenogen).

### Results

Results are shown in Figure 2.

The three biotinylated compounds showed a tendency for inhibition in the same range of parent compound (SST0001), with compound SST0761NA1 that reached statistical significance.

## Claims

1. A compound having the following formula (I) 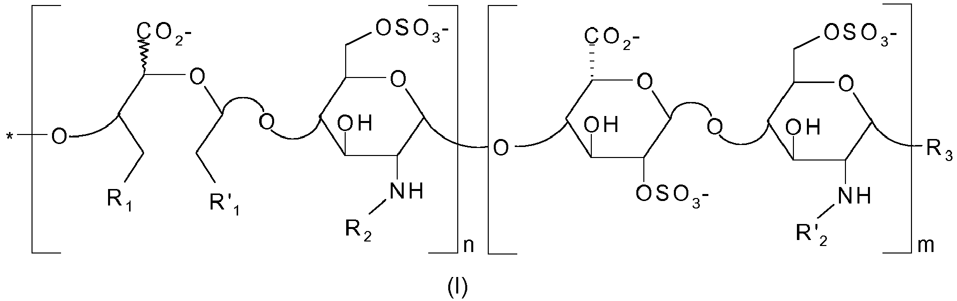 wherein
in each n unit R₁ and R₁' are independently selected from OH and R₄-biotin, wherein R₄ is NH or NH-NH or HN-C₁C₆₀-alkylene-NH,
wherein in said alkylene chain one or more CH₂ group is optionally substituted by one or more of the following groups: O, NH and CO and
in said alkylene chain a hydrogen of one or more CH₂ group is optionally substituted by the side chain of a D or L amino acid;
in each n and m unit R₂ and R₂' are independently selected from an acetyl group, biotin and R₅-biotin, wherein R₅ is CO-C₁C₆₀-alkylene-NH,
wherein in said alkylene chain one or more CH₂ group is optionally substituted by one or more of the following groups: O, NH and CO and
in said alkylene chain a hydrogen of one or more CH₂ group is optionally substituted by the side chain of a D or L amino acid;
R₃ is selected from the group consisting of OH and a disaccharidic moiety of the following formula (II) 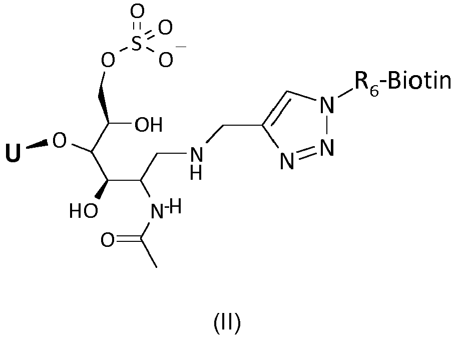
wherein U is selected from 20-sulfated uronic acid and glycol-split uronic acid,
and R₆ is NH or NH-NH or C₁C₆₀-alkylene-NH,
wherein in said alkylene chain one or more CH₂ group is optionally substituted by one or more of the following groups: O, NH and CO and
in said alkylene chain a hydrogen of one or more CH₂ group is optionally substituted by the side chain of a D or L amino acid;
n and m, which can be the same or different, may vary from 1 to 40; the sum of n+m ranges from 6 to 40; the m:n ratio ranges from 10:2 to 1:1, and the units marked with m and n are statistically distributed along the polysaccharide chain and are not necessarily in sequence,
wherein at least one of said groups biotin, R₄-biotin, R₅-biotin and R₆-biotin is present with a substitution degree, expressed in percentage of substituent groups with respect to the total of disaccharide units, comprised between 1 and 50%,
the enantiomers, diastereoisomers, and mixtures thereof,
or a pharmaceutically acceptable salt, hydrate or solvate thereof.

2. The compound according to claim 1 wherein said substitution degree is comprised between 3 and 10%.

3. The compound according to claim 1 or 2 wherein said compound of formula (I) comprises at least one R₅-biotin group in position R₂ and/or R₂' wherein said R₅ comprises a polyethylene glycol (PEG).

4. The compound according to claim 1 or 2 wherein said compound of formula (I) comprises at least one R₄-biotin group in position R₁ and/or R₁' wherein said R₄ is or comprises an amino acid chain.

5. The compound according to claim 1 or 2 wherein in said compound of formula (I) R₃ is a disaccharidic moiety of the above formula (II) comprising at least one R₆-biotin group wherein said R₆ is or comprises a glycine residue and a pentylamine residue in any order.

6. The compound according to anyone of the preceding claims wherein it comprises at least one group R₄-biotin as R₁ and/or R₁', wherein R₄ is or comprises a group selected from the group consisting of an amino group (NH), a hydrazine group (NHNH), a cadaverine residue, a polyethylene glycol (PEG) residue and an amino acid chain comprising two or more amino acids.

7. The compound according to claim 6 wherein said R₄ is or comprises the amino acid chain Gly-Gly-Gly-Ser-Gly-Gly-Gly-Ser-Gly-Gly-Gly.

8. The compound according to anyone of the preceding claims wherein it comprises at least one group R₅-biotin as R₂ and/or R₂', wherein R₅ is or comprises a group selected from the group consisting of a pentylamine residue, a polyethylene glycol (PEG) residue and an amino acid chain comprising two or more amino acids.

9. The compound according to anyone of the preceding claims wherein R₃ is the disaccharidic moiety of the above formula (II) wherein R₆ is or comprises a group selected from the group consisting of an amino group (NH), a hydrazine (NHNH) group, a cadaverine residue, a pentylamine residue, an amino acid residue and an amino acid chain comprising two or more amino acids.

10. The compound according to claim 9 wherein R₆ comprises an amino acid residue or an amino acid chain and a cadaverine residue in any order.

11. The compound according to anyone of the preceding claims which is selected from the group consisting of
N-desulfated N-acetylated glycol split heparin -(GGGS)₃-NH-(CH₂)₅-NH-Biotin with a substitution degree on the R₁ and/or R₁' positions of about 7%;
N-desulfated N-acetylated glycol split heparin -Reducing end-NH-CH₂-1,2,3-Triazole-4, N-Glycine-CONH-(CH₂)₅-NH-Biotin with a substitution degree on the R₃ position of about 2%; and
N-desulfated N-acetylated glycol split heparin -CO-(PEG)₄-CH₂CH₂₋NHCO-(CH₂)₅-NH-Biotin with a substitution degree on the R₂ and/or R₂' positions of about 7%.

12. The compound according to anyone of the preceding claims which is one of the followings:
N-desulfated N-acetylated glycol split heparin -(GGGS)₃-NH-(CH₂)₅-NH-Biotin with a substitution degree on the R₁ and/or R₁' positions of about 3%, and preferably an average molecular weight of about 14000 Da;
N-desulfated N-acetylated glycol split heparin -NH-(CH₂CH₂O)₄-CH₂CH₂-CONH-(CH₂)₅-NH-Biotin with a substitution degree on the R₁ and/or R₁' positions of about 7%, and preferably an average molecular weight of about 9500 Da;
N-desulfated N-acetylated glycol split heparin -NH-(CH₂)₅-NH-Biotin with a substitution degree on the R₁ and/or R₁' positions of about 31%, and preferably an average molecular weight of about 15500 Da;
N-desulfated N-acetylated glycol split heparin -NH-(CH₂)₅-NH-Biotin with a substitution degree on the R₁ and/or R₁' positions of about 12%, and preferably an average molecular weight of about 16000 Da;
N-desulfated N-acetylated glycol split heparin -NH-(CH₂)₅-NH-Biotin with a substitution degree on the R₁ and/or R₁' positions of about 41%, and preferably an average molecular weight of about 17000 Da;
N-desulfated N-acetylated glycol split heparin -NH-(PEG)₄-CH₂CH₂-CONH-(CH₂)₅-NH-Biotin with a substitution degree on the R₁ and/or R₁' positions of about 21%, and preferably an average molecular weight of about 18500 Da;
N-desulfated N-acetylated glycol split heparin -NH-(PEG)₄-CH₂CH₂-CONH-(CH₂)₅-NH-Biotin with a substitution degree on the R₁ and/or R₁' positions of about 28%, and preferably an average molecular weight of about 21000 Da;
N-desulfated N-acetylated glycol split heparin -CO-(CH₂)₅-NH-Biotin with a substitution degree on the R₂ and/or R₂' positions of about 15%, and preferably an average molecular weight of about 9500 Da;
N-desulfated N-acetylated glycol split heparin -CO-(PEG)₄-CH₂CH₂₋NHCO-(CH₂)₅-NH-Biotin with a substitution degree on the R₂ and/or R₂' positions of about 7%, and preferably an average molecular weight of about 8500 Da;
N-desulfated N-acetylated glycol split heparin -Reducing end-NH-CH₂-1,2,3-Triazole-4, N-(CH₂)₅-NH -Biotin, with a substitution degree on the R₃ position of about 4%, and preferably an average molecular weight of about 9500 Da;
N-desulfated N-acetylated glycol split heparin -Reducing end-NH-CH₂-1,2,3-Triazole-4, N-Glycine-CONH-(CH₂)₅-NH-Biotin, with a substitution degree on the R₃ position of about 2%, and preferably an average molecular weight of about 9000 Da;
wherein "Reducing end" means that the linker is on the reducing end of the molecule.

13. The compound according to anyone of claims 1-12 for use as a medicament.

14. The compound according to anyone of claims 1-12 for use in the treatment of a disease which can be improved or prevented by an anti-heparanase activity.

15. The compound for the use according to claim 14 wherein said disease is selected from the group consisting of tumors, primary tumors, metastases, diabetic retinopathies, psoriasis, retrolenticular fibroplasia, restenosis after angioplasty, coronary by-pass, inflammation, sepsis, arthritis, autoimmune diseases, allograft rejection, cardiovascular diseases, fibro-proliferative disease, diseases elicited by abnormal platelet aggregation, diseases elicited by smooth muscle proliferation, Goodpasture syndrome, acute and chronic kidney diseases, glomerulonephritis, membranous nephropathy, diabetic nephropathy, neonatal pulmonary hypertension, asthma, congestive heart failure, adult pulmonary hypertension, renal vascular hypertension, proliferative retinopathies, experimental autoimmune encephalomyelitis, multiple sclerosis, insulin dependent diabetes, inflammatory bowel disease, ulcerative colitis, Crohn's disease, chronic inflammation, bone osteolysis, thrombosis, atherosclerosis, amyloidosis, hereditary multiple exostosis, bladder pain, vulvodynia, acute and chronic pancreatitis, empyema, diabetes, liver fibrosis, kidney fibrosis, peritoneal fibrosis and aging.

16. A pharmaceutical composition containing as active ingredient a compound according to anyone of claims 1-12 and at least one pharmaceutically acceptable vehicle and/or excipient.

17. The pharmaceutical composition according to claim 16 wherein said composition further comprises one or more further active ingredients.

18. The pharmaceutical composition according to claim 17 wherein said further active ingredient is a chemotherapeutic agent.

19. The pharmaceutical composition according to claim 18 wherein said chemotherapeutic agent is selected from the group consisting of cytotoxic agents, proteasome inhibitors, immunomodulatory drugs, topoisomerase inhibitors, kinase inhibitors and monoclonal antibodies, in particular directed towards highly expressed tumor associated antigens.

20. The pharmaceutical composition according to claim 19 wherein said chemotherapeutic agent is a cytotoxic agent selected from the group consisting of melphalan, irinotecan, rituximab and lapatinib; or is a proteasome inhibitor selected from the group consisting of bortezomib, carfilzomib and ixazomib; or is an immunomodulatory agent selected from the group consisting of thalidomide, lenalidomide and pomalidomide.
